# DEMANDE DE BREVET EUROPEEN

(11) **EP 0 611 766 A1**
(43) Date de publication de la demande: **24.08.1994**
(21) Numéro de dépôt: 94400350.8
(22) Date de dépôt: 17.02.1994
(51) Int. Cl.: C07D 417/12, C07D 417/14, C07D 277/40, C07D 417/04, A61K 31/425

(54) **Dérivés de 2-amido-thiazoles polysubstitués, procédé de préparation, composition pharmaceutique et utilisation de ces dérivés pour la préparation d'un médicament**

(30) Priorité: 19.02.1993 FR 9301941
(71) Demandeur: SANOFI, F-75008 Paris (FR)
(72) Inventeur: Boigegrain, Robert, F-34820 Assas (FR); Brodin, Roger, F-34070 Montpellier (FR); Frehel, Daniel, F-31000 Toulouse (FR); Gully, Danielle, F-31600 Saubens (FR); Molimard, Jean-Charles, F-34980 Saint-Gely du Fesc (FR); Olliero, Dominique, F-34100 Montpellier (FR)
(74) Mandataire: Le Guen, Gérard

(57) **Abrégé**

L'invention a pour objet l'utilisation d'un composé de formule 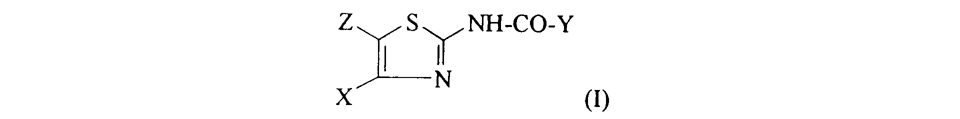
dans laquelle Y représente un groupe 3-quinoléinyle ou un groupe 2-indolyle de formule : 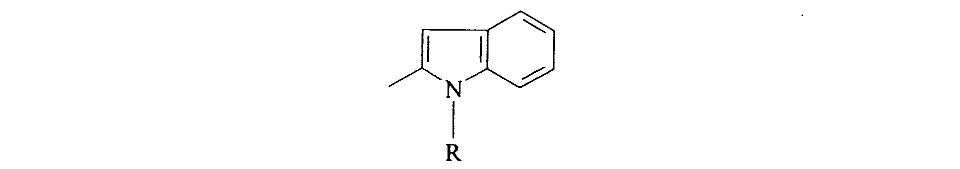
dans laquelle :
- R est l'hydrogène, un groupe acétyle ou un groupe CH₂COOR', R' étant l'hydrogène ou un alkyle en C₁-C₄ ;
- X est un radical (hétéro)aryle choisi parmi les groupes 4-chloro-2,6-diméthoxyphényle, 2,6-diméthoxy-4-méthylphényle, 2,4,5-triméthoxyphényle, 4-méthyl-2,3,6-triméthoxyphényle, 2,6-diméthoxy-4-éthylphényle, 2,4,6-triméthoxy-5-chlorophényle, 2,4,6-triméthoxy-3-pyridinyle, 2,4-diméthoxy-6-méthyl-3-pyridinyle, 6-chloro-2,4-diméthoxy-5-pyrimidinyle, 2,4,6-triméthoxy-5-pyrimidinyle, 5-chloro-2,4-diméthoxyphényle, S-chloro-2-méthoxy-4-méthylphényle, 2,5-diméthoxy-4-méthylphényle, 4-trifluorométhyl-2,6-diméthoxyphényle, 2,4-diméthoxy-5-méthylphényle, 5-éthyl-2,4-diméthoxyphényle, 2,4-diméthoxyphényle ;
- Z représente H, un alkyle en C₁-C₄ ou un benzyle ;

avec la limitation que Z est obligatoirement l'hydrogène, lorsque X est un radical phényle substitué simultanément aux positions 2 et 6 ou lorsque X est un radical 3-pyridinyle substitué simultanément aux positions 2 et 4 ou lorsque X est un radical 5-pyrimidinyle substitué simultanément aux positions 4 et 6.

## Description

La présente invention concerne l'utilisation de dérivés du thiazole pour la préparation de médicaments. Elle concerne également de nouveaux dérivés du thiazole, un procédé pour leur préparation et les médicaments en contenant.

Plus particulièrement, la présente invention a pour objet de nouveaux agonistes des récepteurs de la cholécystokinine (CCK) sur le test de l'amylase pancréatique.

La CCK est un peptide largement distribué dans le cerveau, notamment dans le cortex, le striatum, l'hippocampe, le tegmentum ventral, le septum et l'hypothalamus.

La CCK est également secrétée au niveau périphérique par l'intestin grêle, son action se manifeste notamment par la stimulation de la contraction vésiculaire, une augmentation de la sécrétion biliaire, le contrôle de la sécrétion enzymatique pancréatique, une action sur les contractions gastriques, une action sur la motilité intestinale. Elle pourrait agir dans certains cas sur la pression artérielle et influencer les systèmes immunitaires.

La CCK coexiste dans certains neurones centraux avec la dopamine. Elle intervient également dans des mécanismes impliquant l'acétylcholine, le gaba (acide 4-aminobutyrique), la sérotonine, les opioïdes, la somatostatine, la substance P et des canaux ioniques.

Son administration provoque des modifications physiologiques : ptose palpébrale, hypothermie, hyperglycémie, catalepsie, et comportementales : hypolocomotricité, diminution de l'exploration, analgésie, modification de la faculté d'apprentissage, modification du comportement sexuel et satiété.

Un agoniste des récepteurs de la CCK peut donc être utilisé comme médicament dans le traitement de certains troubles du comportement alimentaire, de l'obésité, du diabète, des troubles du comportement émotionnel, sexuel et mnésique, de la schizophrénie, des psychoses, de la maladie de Parkinson et de divers troubles de la sphère gastrointestinale (Drugs of the future, 1992, 17 (3), 197-206).

Des agonistes du récepteur de la CCK sont décrits dans la littérature. Par exemple, certains produits ayant de telles propriétés sont décrits dans EP-A-0383690, WO 90/06937 et EP-A-0376849.

La demande de brevet EP-A-0432040 décrit des acylaminothiazoles ayant une affinité pour le récepteur CCK A et le récepteur CCK B. Certains des composés revendiqués dans la demande EP-A-0432040 ont été décrits notamment comme des antagonistes des récepteurs CCK A et CCK B.

On a maintenant trouvé de façon surprenante qu'une série d'acylaminothiazoles, dont certains sont compris dans EP-A-0432040, possèdent une puissante activité agoniste des récepteurs de la CCK et sont donc utiles pour la préparation de médicaments CCK-agonistes.

Ainsi, selon un de ses aspects, l'invention a pour objet l'utilisation de N-thiazol-2-ylindolecarboxamides ou de N-thiazol-2-ylquinoléinecarboxamide de formule : 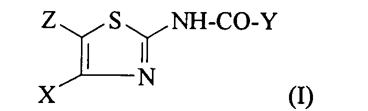
dans laquelle Y représente un groupe 3-quinoléinyle ou un groupe 2-indolyle de formule : 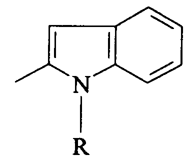
dans laquelle :
- R est l'hydrogène, un groupe acétyle ou un groupe CH₂COOR', R' étant l'hydrogène ou un alkyle en C₁-C₄ ;
- X est un radical (hétéro)aryle choisi parmi les groupes 4-chloro-2,6-diméthoxyphényle, 2,6-diméthoxy-4-méthylphényle, 2,4,5-triméthoxyphényle, 4-méthyl-2,3 ,6-triméthoxyphényle, 2,6-diméthoxy-4-éthylphényle, 2,4,6-triméthoxy-5-chlorophényle, 2,4,6-triméthoxy-3-pyridinyle, 2,4-diméthoxy-6-méthyl-3-pyridinyle, 6-chloro-2,4-diméthoxy-5-pyrimidinyle, 2,4,6-triméthoxy-5-pyrimidinyle, 5-chloro-2,4-diméthoxyphényle, 5-chloro-2-méthoxy-4-méthylphényle, 2,5-diméthoxy-4-méthylphényle, 4-trifluorométhyl-2,6-diméthoxyphényle, 2,4-diméthoxy-5-méthylphényle, 5-éthyl-2,4-diméthoxyphényle,2,4-diméthoxyphényle ;
- Z représente H, un alkyle en C₁-C₄ ou un benzyle ;
   avec la limitation que Z est obligatoirement l'hydrogène, lorsque X est un radical phényle substitué simultanément aux positions 2 et 6 ou lorsque X est un radical 3-pyridinyle substitué simultanément aux positions 2 et 4 ou lorsque X est un radical 5-pyrimidinyle substitué simultanément aux positions 4 et 6 ;
   ainsi que de leurs sels pharmaceutiquement acceptables et de leurs solvates,
   pour la préparation de médicaments destinés à combattre les maladies dont le traitement nécessite une stimulation par agonisme total ou partiel des récepteurs de la cholécystokinine.

Parmi les composés de formule (I) ci-dessus, certains ne sont pas décrits dans la littérature et constituent donc un autre objet de la présente invention.

Ainsi, selon un autre de ses aspects, l'invention concerne de nouveaux dérivés d'acylaminothiazoles de formule : 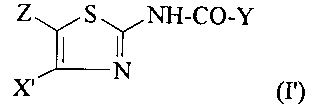
dans laquelle Y représente un groupe 3-quinoléinyle (a) ou un groupe 2-indolyle (b) de formule : 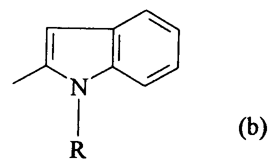
dans laquelle :
- R est l'hydrogène, un groupe acétyle ou un groupe CH₂COOR', R' étant l'hydrogène ou un alkyle en C₁-C₄ ;
- X' est un radical (hétéro)aryle choisi parmi les groupes 4-chloro-2,6-diméthoxyphényle, 2,6-diméthoxy-4-méthylphényle, 2,4,5-triméthoxyphényle, 4-méthyl-2,3,6-triméthoxyphényle, 2,4,6-triméthoxy-5-chlorophényle, 2,4,6-triméthoxy-3-pyridinyle, 2,4,6-triméthoxy-5-pyrimidinyle, 2,4-diméthoxy-6-méthyl-3-pyridinyle, 6-chloro-2,4-diméthoxy-5-pyrimidinyle, 5-chloro-2,4-diméthoxyphényle, 5-chloro-2-méthoxy-4-méthylphényle, 2,5-diméthoxy-4-méthylphényle, 4-trifluorométhyl-2,6-diméthoxyphényle, 2,4-diméthoxy-5-méthylphényle, 5-éthyl-2,4-diméthoxyphényle ;
- Z représente H, un alkyle en C₁-C₄ ou un benzyle ;
   avec la limitation que Z représente H lorsque X est un radical phényle substitué simultanément aux positions 2 et 6 ou lorsque X est un radical 3-pyridinyle substitué simultanément aux positions 2 et 4 ou lorsque X est un radical 5-pyrimidinyle substitué simultanément aux positions 4 et 6 ;
   ainsi que leurs sels et leurs solvates.

Les sels d'addition de ces composés sont ceux obtenus avec des acides et des bases minérales ou organiques : les sels non toxiques pharmaceutiquement acceptables sont préférés mais d'autres sels utilisables pour isoler ou purifier les composés de formule (I') sont aussi un objet de l'invention.

Les composés de formule (I'), dans laquelle Y représente un groupe (b) où R est l'hydrogène ou un groupe CH₂COOH, sont particulièrement avantageux.

Les composés de formule (I'), dans laquelle Z représente un hydrogène ou un méthyle, sont particulièrement avantageux.

Les composés de formule (I'), dans laquelle Y représente un radical (b) où R est l'hydrogène ou un groupe CH₂COOH, Z représente un hydrogène ou un méthyle et X' représente un radical aryle choisi parmi les groupes 4-chloro-2,6-diméthoxyphényle, 5-chloro-2,4-diméthoxyphényle, 5-chloro-2-méthoxy-4-méthylphényle, 2,6-diméthoxy-4-méthylphényle, 2,4-diméthoxy-5-méthylphényle et 2,4,5-triméthoxyphényle (Z étant obligatoirement un hydrogène lorsque X' représente un groupe 4-chloro-2,6-diméthoxyphényle ou 2,6-diméthoxy-4-méthylphényle), sont préférés.

Parmi les composés de formule (I') ci-dessus,
- le N-[4-(4-chloro-2,6-diméthoxyphényl)thiazol-2-yl]-1-(carboxyméthyl)indole-2-carboxamide, ses sels et solvates pharmaceutiquement acceptables, notamment le chlorhydrate,
- le N-[4-(5-chloro-2,4-diméthoxyphényl)thiazol-2-yl]-1H-indole-2-carboxamide, ses sels et solvates pharmaceutiquement acceptables,
- le N-[4-(5-chloro-2,4-diméthoxyphényl)-5-méthylthiazol-2-yl]-1H-indole-2-carboxamide, ses sels et solvates pharmaceutiquement acceptables,
- le N-[4-(5-chloro-2,4-diméthoxyphényl)-5-méthylthiazol-2-yl]-1-(carboxyméthyl)-indole-2-carboxamide, ses sels et solvates pharmaceutiquement acceptables, notamment le trifluoroacétate,
- le N-[4-(5-chloro-2-méthoxy-4-méthylphényl)thiazol-2-yl]-1H-indole-2-carboxamide, ses sels et solvates pharmaceutiquement acceptables,
- le N-[4-(2,6-diméthoxy-4-méthylphényl)thiazol-2-yl]-1H-indole-2-carboxamide, ses sels et solvates pharmaceutiquement acceptables, notamment le chlorhydrate monohydrate,
- le N-[4-(2,4-diméthoxy-5-méthylphényl)-5-méthylthiazol-2-yl]-1H-indole-2-carboxamide, ses sels et solvates pharmaceutiquement acceptables,
- le N-[4-(2,4,5-triméthoxyphényl)-5-méthylthiazol-2-yl]-1H-indole-2-carboxamide, ses sels et solvates pharmaceutiquement acceptables,
   sont particulièrement préférés.

L'invention a également pour objet un procédé pour la préparation des composés de formule (I') caractérisé en ce qu'on condense un acide de formule (II)

Y' - COOH (II)

dans laquelle Y' représente un radical 3-quinoléinyle (a) 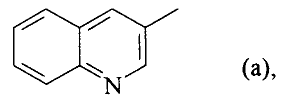
un radical 2-indolyle (b°) 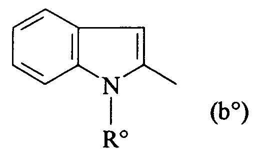
ou un radical 2-indolinyle (c°) 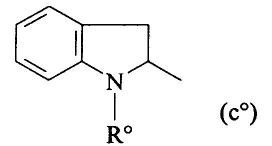
R° étant un groupe N-protecteur ou un groupe CH₂COOR'', où R'' est un alkyle en C₁-C₄ ; ou bien un dérivé fonctionnel dudit acide (II) sur un 2-aminothiazole de formule : 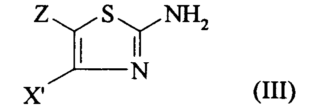
dans laquelle X' et Z sont tels que définis ci-dessus, en présence d'une base pour obtenir un composé de formule (I'') : 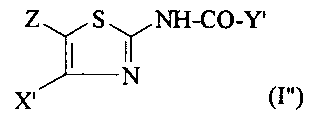
dans laquelle X' et Z sont tels que définis ci-dessus et Y' est un des radicaux (a), (b°) ou (c°) tels que définis ci-dessus, puis,
- lorsque dans le composé (I''), Y' est un radical (b°), on soumet éventuellement le produit ainsi obtenu de formule (I''b°) : 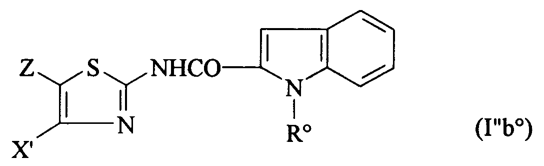 à une N-déprotection ou à une saponification ou à une hydrolyse acide ;
- lorsque dans le composé (I''), Y' est un radical (c°), on soumet le produit ainsi obtenu de formule : 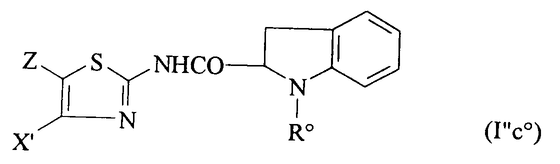 à une déshydrogénation, éventuellement précédée d'une N-déprotection, d'une saponification, ou d'une hydrolyse acide pour obtenir un composé de formule (I') dans laquelle Y est un radical (b) où R est l'hydrogène ou un groupe CH₂COOR', Z, X' et R' étant tels que définis précédemment;
   et on isole le produit de formule (I') tel quel ou sous forme d'un de ses solvates ou de ses sels pharmaceutiquement acceptables.

Comme dérivé fonctionnel de l'acide (II) on peut utiliser l'acide même, éventuellement activé, son anhydride, un de ses anhydrides mixtes ou un de ses esters activés.

La condensation de l'aminothiazole (III) avec l'acide (II) sous forme d'ester activé, préparé par exemple par action du 1-hydroxybenzotriazole sur l'acide en présence de dicyclohexylcarbodiimide selon le mode opératoire décrit dans J. Am. Chem. Soc. 1971, *93*, 6318-6319, ou par action de l'hexafluorophosphate de 1-benzotriazolyloxytrisdiméthylaminophosphonium (BOP) selon le mode opératoire décrit dans Synthesis, 1976, 751-752, peut être effectuée dans un solvant dont la nature est choisie selon la solubilité des composés et le type d'activation de la fonction acide, de préférence en présence d'une base, par exemple une amine tertiaire telle que la triéthylamine ; la réaction est en général effectuée à une température comprise entre 0°C et 30°C.

Par la première étape du procédé selon l'invention, on obtient un composé de formule (I'') où Z, X' et Y' sont tels que définis ci-dessus. Lorsque, dans le composé de formule (I''), Y' représente
- soit un radical (a),
- soit un radical (b°) dans lequel R° est un groupe CH₂COOR'',
   ledit composé peut également représenter le produit final de formule (I') où Y représente soit un radical (a), soit un radical (b) dans lequel R est un groupe CH₂COOR' dans lequel R' est un alkyle en C₁-C₄, Z et X' étant tels que définis précédemment.

Lorsque dans le composé de formule (I'') Y' représente un groupe (b°) dans lequel R° est un groupe N-protecteur ou un groupe CH₂COOR'', ledit composé peut être N-déprotégé pour obtenir des composés de formule (I') dans laquelle Y représente un groupe (b) où R est l'hydrogène ou bien il peut être soumis à une saponification ou à une hydrolyse acide pour obtenir un composé de formule (I') dans laquelle Y représente un groupe (b) où R est un groupe CH₂COOH.

Lorsque dans le composé de formule (I'') Y' représente un groupe (c°), ledit composé est soumis aux déprotections auxquelles s'ajoute une déshydrogénation.

Les acides Y'COOH dans lesquels R°, dans les radicaux (b°) et (c°), est un groupe protecteur acyle tel qu'acétyle peuvent être préparés par action du chlorure d'acétyle ou de l'anhydride acétique par exemple, sur Y'COOH dans lequel R° = H et en présence d'un équivalent de triéthylamine ou de 4-diméthylaminopyridine, par exemple dans le dichlorométhane.

Lorsque le dérivé fonctionnel de l'acide II est un anhydride mixte, celui-ci peut être préparé par action d'un chloroformiate d'alkyle sur l'acide, en présence d'une base, généralement une amine tertiaire telle que la triéthylamine; cette réaction est le plus souvent effectuée dans un solvant tel que le dichlorométhane, le dichloroéthane ou le chloroforme.

Lorsqu'on veut préparer un indole-2-carboxamide de formule (I') où R est l'hydrogène, R° représente un groupe N-protecteur dans les radicaux (b°) et (c°).

Ainsi les dérivés (I') dans lesquels Y représente : 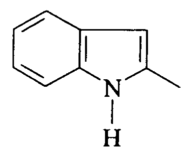
peuvent être préparés à partir des composés obtenus par condensation de l'aminothiazole (III) avec un dérivé fonctionnel de l'acide indole-2-carboxylique de formule : 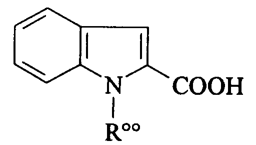
dans laquelle R°° représente un groupe N-protecteur habituellement utilisé pour la protection des groupes NH₂ dans les réactions de condensation des acides aminés, tel que : -COOC(CH₃)₃ ; -COOCH₂C₆H₅; -CO-CH₃ ; le groupe N-protecteur peut ensuite être éliminé par les méthodes de déprotection classiques.

Les mêmes composés peuvent être aussi préparés à partir des composés obtenus par condensation de l'aminothiazole (III) avec des dérivés de l'acide indoline-2-carboxylique de formule : 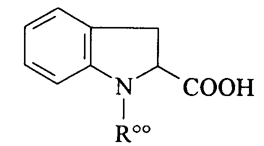
dans laquelle R°° est un groupe N-protecteur tel que -COOC(CH₃)₃ pour obtenir le composé de formule : 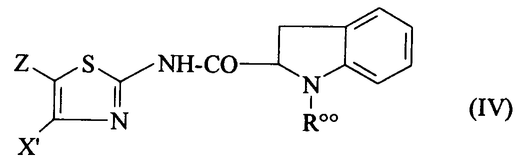
le groupe R°° pouvant être éliminé du composé (IV) par action d'un acide fort en milieu anhydre tel que l'acide trifluoroacétique dans le dichlorométhane ou l'acide chlorhydrique dans l'éther éthylique.

Le composé ainsi obtenu est ensuite déshydrogéné.

La réaction est effectuée par action sur le reste indoline des réactifs déshydrogénants classiques, tel que la 2,3,5,6-tétrachloro-1,4-benzoquinone, la 2,3-dichloro-5,6-dicyano-1,4-benzoquinone (DDQ) ou le cyclohexène en présence de Palladium dans des solvants inertes à haut point d'ébullition tels que le diphényléther, le xylène, le 1,2-diméthoxyéthane ou le 2-méthoxyéthyléther à température élevée, et de préférence à la température de reflux du solvant.

Lorsque le groupe protecteur représenté par R°° est acétyle, il peut également constituer le groupe R du substituant Y du produit final de formule (I').

L'hydrolyse de l'ester alkylique en C₁-C₄ du groupement R°, pour l'obtention des produits de formule (I') où Y représente CH₂COOH, est effectuée soit en milieu acide, soit de préférence en milieu basique, par exemple par action d'une base minérale, tel qu'un hydroxyde alcalin, en milieu hydroalcoolique.

Les aminothiazoles de formule 2-amino-4-(2,4,5-triméthoxyphényl)-5-méthylthiazole et 2-amino-4-(2,4,5-triméthoxyphényl)thiazole sont décrits dans Rev. Latinoam. Quim., 1990, 21 (3-4), 102-105.

Les aminothiazoles de formule : 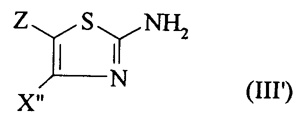
dans laquelle :
- X'' représente un radical(hétéro)aryle choisi parmi les groupes 4-chloro-2,6-diméthoxyphényle, 2,6-diméthoxy-4-méthylphényle, 4-méthyl-2,3,6-triméthoxyphényle, 2,4,6-triméthoxy-5-chlorophényle, 2,4,6-triméthoxy-3-pyridinyle, 2,4,6-triméthoxy-5-pyrimidinyle, 2,4-diméthoxy-6-méthyl-3-pyridinyle, 6-chloro-2,4-diméthoxy-5-pyrimidinyle, 5-chloro-2,4-diméthoxyphényle, 5-chloro-2-méthoxy-4-méthylphényle, 2,5-diméthoxy-4-méthyl-phényle, 4-trifluorométhyl-2,6-diméthoxyphényle, 2,4-diméthoxy-5-méthylphényle, 5-éthyl-2,4-diméthoxyphényle ;
- Z représente H, un alkyle en C₁-C₄ ou un benzyle ;
   avec la limitation que Z représente H lorsque X'' est un radical phényle substitué simultanément aux positions 2 et 6 ou lorsque X'' est un radical 3-pyridinyle substitué simultanément aux positions 2 et 4 ou lorsque X'' est un radical 5-pyrimidinyle substitué simultanément aux positions 4 et 6 ;
   sont nouveaux et font partie de l'invention.

Parmi les composés de formule (III') ci-dessus,
- le 2-amino-4-(4-chloro-2,6-diméthoxyphényl)thiazole,
- le 2-amino-4-(5-chloro-2,4-diméthoxyphényl)thiazole,
- le 2-amino-4-(5-chloro-2,4-diméthoxyphényl)-5-méthylthiazole,
- le 2-amino-4-(5-chloro-2-méthoxy-4-méthylphényl)thiazole,
- le 2-amino-4-(2,6-diméthoxy-4-méthylphényl)thiazole,
- le 2-amino-4-(2,4-diméthoxy-5-méthylphényl)-5-méthylthiazole,
   sont particulièrement préférés.

Ils peuvent être préparés selon l'un des procédés décrits, notamment dans Bull. Soc. Chim. (C), 1963, 2498-2503.

De façon générale, on fait réagir la thiourée avec une cétone alpha-halogénée, et de préférence alpha-chlorée, selon le schéma réactionnel suivant : 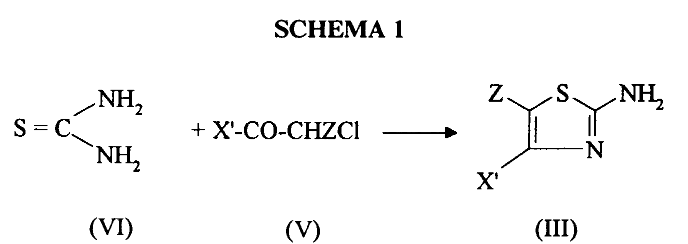
X' et Z ayant la même signification que ci-dessus.

Les cétones (V) peuvent être obtenues par exemple :
(1) par réaction de Friedel et Crafts : 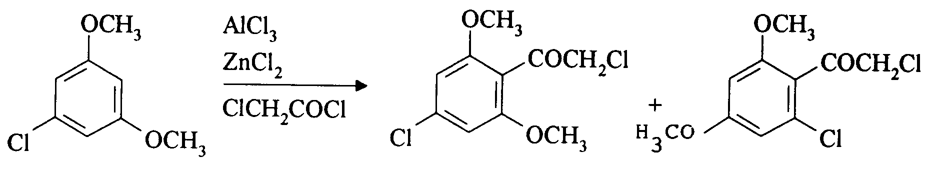 selon Chem. Pharm. Bull., 1991, *39*, *9*, 2400-2407.
(2) par réaction de lithiation : 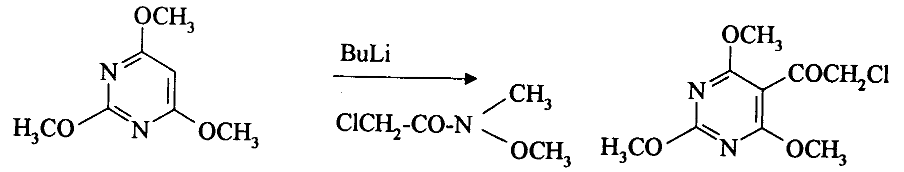 selon EP-A-0432040.

Les aminothiazoles (III) peuvent également être préparés en une seule étape en utilisant la réaction de Hoesch (selon Dubois, Organic Reactions, 1949, *5*, 387 ou selon Satchell et al., The Chemistry of the Carbonyl Group, ed. S. Patai, Interscience, 1966, *1*, *5*, 233-302) sur un dérivé benzénique substitué suivie de la cyclisation avec la thiourée.

Les aminothiazoles (III) peuvent également être préparés en une étape à partir de cétones aromatiques, selon le schéma réactionnel suivant :

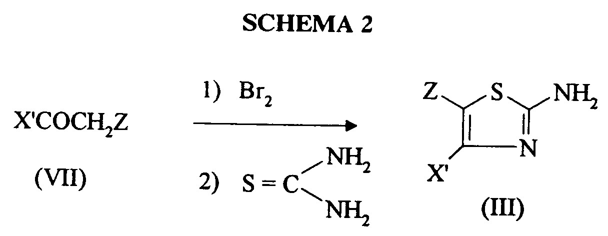

Les cétones aromatiques (VII) de départ sont préparées par réaction de Friedel et Crafts à partir des dérivés X'H.

Les dérivés X'H sont connus ou préparés par des méthodes connues.

Certains des acides Y'COOH sont connus et même commerciaux ; les autres sont préparés en utilisant les méthodes connues pour des molécules analogues. Ils sont tous illustrés dans EP-A-0432040.

Ainsi, les acides indole-2-carboxyliques de formule : 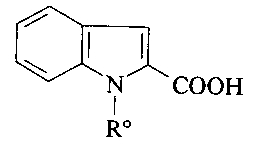
dans laquelle R° représente un groupe alcoxycarbonylméthyle en C₁-C₄ peuvent être préparés à partir des acides indole-2-carboxyliques commerciaux ou obtenus par des procédés classiques en suivant le SCHEMA 3 ci-dessous, 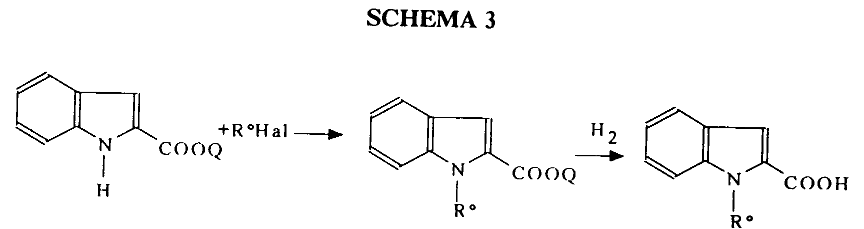
dans lequel Hal représente un atome d'halogène et Q représente le groupe benzyle.

Les esters benzyliques de départ du SCHEMA 3 sont préparés par action de l'acide correspondant sur l'alcool benzylique, en présence de l'un des agents d'activation de la fonction acide couramment utilisés en synthèse peptidique et tel que décrit dans EP-A-0432040.

Les sels des composés de formule (I') avec des acides ou des bases organiques ou minérales sont préparés de la façon habituelle par introduction de l'acide, ou de la base dans une solution du composé de formule (I'). Le sel est isolé, selon ses caractéristiques de solubilité, après évaporation du solvant ou addition d'un non-solvant.

L'invention a également pour objet selon un autre de ses aspects des compositions pharmaceutiques comprenant les composés (I') ci-dessus.
Plus généralement, les composés de formule (I) ont fait l'objet d'études de binding *in* *vitro* concernant les récepteurs CCK.

Une étude de l'effet agoniste des composés sur la sécrétion d'amylase a été réalisée comme suit. Les acini pancréatiques sont obtenus par digestion enzymatique (collagénase) de pancréas de rat soumis à un jeûne de 18 heures. Des aliquotes (485 µl) sont incubées à 37°C pendant 30 minutes en présence de concentrations croissantes d'agoniste selon Jensen et al., J. Biol. Chem., 1982, *257* (10), 5554. L'incubation est arrêtée par une centrifugation de 15 secondes. Le surnageant est conservé dans un bain de glace pour mesurer, le taux d'amylase selon la technique de Ceska et al., Clin. Chim. Acta, 1969, *26*, 437 (réactif phadebas®: test amylase commercialisé par pharmacia diagnostic). Les composés à tester sont dissous dans du diméthylsulfoxide puis dans un tampon d'incubation.

Les composés de formule (I) se comportent comme des agonistes des récepteurs CCK avec des DE₅₀ (dose efficace induisant 50% de la sécrétion d'amylase comparé à l'effet maximum produit en présence de CCK) de l'ordre de 10⁻⁹Mol

Une étude de l'effet agoniste CCK des composés sur la consommation alimentaire a été réalisée comme suit. Les rats mâles (200-240 g) Sprague Dawley (Charles River, France), sont isolés 10 jours avant l'expérience, et soumis chaque jour successivement à 18 heures de jeûne et 6 heures d'alimentation : la nourriture est disponible de 10 heures à 16 heures, l'eau est disponible ad libitum. Le jour de l'expérience, les produits (en suspension dans une solution de méthylcellulose à 0,6 %) ou le véhicule sont administrés par voie intra-péritonéale. Trente minutes après le traitement (à 10 heures) une quantité connue de nourriture est introduite dans la cage : on mesure la consommation alimentaire 1 heure et 3 heures après.

Les composés de formule (I) diminuent la prise de nourriture et se comportent donc comme des agonistes des récepteurs CCK (Gibbs J. et al., J. Comp. Physiol. Psychol., 1973, 84, 488-495) en particulier :
le N-[4-(5-chloro-2,4-diméthoxyphényl)thiazol-2-yl]-1H-indole-2-carboxamide,
le N-[4-(5-chloro-2,4-diméthoxyphényl)-5-méthylthiazol-2-yl]-1H-indole-2-carboxamide,
le trifluoroacétate de N-[4-(5-chloro-2,4-diméthoxyphényl)-5-méthylthiazol-2-yl]-1-(carboxyméthyl)indole-2-carboxamide,
le N-[4-(5-chloro-2-méthoxy-4-méthylphényl)thiazol-2-yl]-1H-indole-2-carboxamide,
sont actifs à la dose de 3 mg/kg, dose à laquelle ils réduisent la consommation alimentaire de 30 à 40 % par rapport à un animal témoin.

Par conséquent, les composés de formule (I) sont utilisés, en tant qu'agoniste des récepteurs de la CCK, pour la préparation de médicaments destinés à combattre les maladies dont le traitement nécessite une stimulation par agonisme total ou partiel des récepteurs de la chlolecystokinine, plus particulièrement pour la fabrication de médicaments destinés au traitement de certains troubles du comportement alimentaire, de l'obésité, du diabète, des troubles du comportement émotionnel, sexuel et mnésique, des psychoses et notamment la schizophrénie, de la maladie de Parkinson et de divers troubles de la sphère gastrointestinale.

Les composés de formule (I) sont peu toxiques ; leur toxicité est compatible avec leur utilisation comme médicament pour le traitement des troubles et des maladies ci-dessus.

Les nouveaux composés de formule (I'), peuvent être formulés dans des compositions pharmaceutiques pour l'administration aux mammifères, y compris l'homme, pour le traitement des maladies susdites.

La posologie, variable selon le traitement et selon l'affection en cause peut s'échelonner, par exemple, entre 0,05 et 100 mg par jour chez l'adulte par voie orale.

La présente invention a également pour objet les compositions pharmaceutiques qui contiennent à titre de principe actif un des composés ci-dessus. Ces compositions sont réalisées de façon à pouvoir être administrées par la voie digestive ou parentérale.

Dans les compositions pharmaceutiques de la présente invention pour l'administration orale, sublinguale, sous-cutanée, intramusculaire, intraveineuse, transdermique, locale ou rectale, l'ingrédient actif peut être administré sous formes unitaires d'administration, en mélange avec des supports pharmaceutiques classiques, aux animaux et aux êtres humains. Les formes unitaires d'administration appropriées comprennent les formes par voie orale telles que les comprimés, les gélules, les poudres, les granules et les solutions ou suspensions orales, les formes d'administration sublinguale et buccale, les formes d'administration sous-cutanée, intramusculaire, intraveineuse, intranasale ou intraoculaire et les formes d'administration rectale.

Lorsque l'on prépare une composition solide sous forme de comprimés, on mélange l'ingrédient actif principal avec un véhicule pharmaceutique tel que la gélatine, l'amidon, le lactose, le stéarate de magnésium, le talc, la gomme arabique ou analogues. On peut enrober les comprimés de saccharose ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant l'ingrédient actif avec un diluant et en versant le mélange obtenu dans des gélules molles ou dures.

Une préparation sous forme de sirop ou d'élixir peut contenir l'ingrédient actif conjointement avec un édulcorant, acalorique de préférence, du méthylparaben et du propylparaben comme antiseptique, ainsi qu'un agent donnant du goût et un colorant approprié.

Les poudres ou les granules dispersibles dans l'eau peuvent contenir l'ingrédient actif en mélange avec des agents de dispersion ou des agents mouillants, ou des agents de mise en suspension, comme la polyvinylpyrrolidone, de même qu'avec des édulcorants ou des correcteurs du goût.

Pour une administration rectale, on recourt à des suppositoires qui sont préparés avec des liants fondant à la température rectale, par exemple du beurre de cacao ou des polyéthylèneglycols.

Pour une administration parentérale, intranasale ou intraoculaire, on utilise des suspensions aqueuses, des solutions salines isotoniques ou des solutions stériles et injectables qui contiennent des agents de dispersion et/ou des agents mouillants pharmacologiquement compatibles, par exemple le propylèneglycol ou le butylèneglycol.

Le principe actif peut être formulé également sous forme de microcapsules, éventuellement avec un ou plusieurs supports ou additifs.

Le principe actif peut être également présenté sous forme de complexe avec une cyclodextrine, par exemple α, β ou γ cyclodextrine, 2-hydroxypropyl-β-cyclodextrine ou méthyl-β-cyclodextrine.

La composition peut être sous forme de dose unitaire comprenant de 0,05 à 100 mg de principe actif.

Dans ce qui suit, on décrit des exemples de mise en oeuvre de l'invention, ainsi que les procédés de préparation de certains intermédiaires de synthèse de formule X'H, (V), (VII), (III) et (II). Les points de fusion, indiqués ont été déterminés en capillaire. Les spectres de résonance magnétique nucléaire, ont été enregistrés par rapport au tétraméthylsilane.

Dans les préparations et dans les exemples, les abréviations suivantes sont utilisées.
DCM : dichlorométhane
Ether : éther diéthylique
Ether iso : éther diisopropylique
CCl₄ : tétrachlorure de carbone
MeOH : méthanol
EtOH : éthanol
AcOEt : acétate d'éthyle
DMF : diméthylformamide
THF: tétrahydrofurane
CHCl₃ : chloroforme
AlCl₃ : chlorure d'aluminium
ZnCl₂ : chlorure de zinc
TiCl₄ : chlorure de titane
HCl : acide chlorhydrique
H₂SO₄ : acide sulfurique
TFA : acide trifluoroacétique
KHSO₄ : hydrogénosulfate de potassium
NaOH : soude
silice H : gel de silice 60 H commercialisé par MERCK (DARMSTAD)
tBU *: tert*-butyle
F: point de fusion
T_{eb} : température d'ébullition
TA : température ambiante
RMN : résonnance magnétique nucléaire
s : singulet
se : singulet élargi
m : massif

### PREPARATION I. Composés X'H.

A) 2,4,6-Triméthoxypyrimidine.
   On prépare ce composé selon le mode opératoire décrit dans J. Am. Chem. Soc., 1932, 54, 727-733.
B) 2,4-Diméthoxy-6-méthylpyridine.
   On prépare tout d'abord la 1,2-dihydro-4-hydroxy-6-méthyl-2-oxopyridine selon le mode opératoire décrit dans J. Heterocycl. Chem., 1975, 12 (5), 963-967.
   On chauffe à 120°C pendant 2 heures et 30 minutes un mélange de 7,51 g du composé obtenu précédemment et 75 ml d'oxychlorure de phosphore. On abandonne une nuit à TA, évapore sous vide le mélange réactionnel, reprend le résidu dans de la glace, ajoute une solution saturée d'hydrogénocarbonate de sodium jusqu'à pH = 10, extrait à l'éther, sèche sur sulfate de sodium et évapore sous vide le solvant. On obtient 9,7 g de 2,4-dichloro-6-méthylpyridine sous forme d'huile.
   On chauffe, pendant 36 heures, à une température comprise entre 130 et 140°C, dans un réacteur, sous une pression de 5 bars un mélange de 9,7 g du composé obtenu précédemment, 7,13 g de méthylate de sodium dans 15 ml de MeOH. Après refroidissement, on ajoute 200 ml d'éther, filtre et évapore sous vide le filtrat. On chromatographie sur silice en éluant par du DCM. On obtient 4 g du produit monométhoxylé que l'on remet en réaction. On chauffe 20 heures à une température comprise entre 132° et 140°C, dans un réacteur sous une pression de 5 bars un mélange de 4 g du produit précédent avec une solution de méthylate de sodium préparée à partir de 0,7 g de sodium et 15 ml de MeOH. Après refroidissement, on ajoute 200 ml d'éther, filtre et évapore à pression atmosphérique le filtrat. On distille le résidu sous vide et obtient 2,5 g du produit attendu, T_{Eb} = 101-103°C sous 0,02 bar.
C) 1-Chloro-2,4-diméthoxybenzène.
   A une solution de 20 g de 4-chlororésorcinol dans 200 ml d'EtOH on ajoute 82,6 g d'une solution à 50 % en poids d'hydroxyde de césium dans l'eau. On évapore sous vide, reprend le résidu dans l'isopropanol, évapore à nouveau sous vide et répète trois fois cette opération. On met le sel de césium ainsi obtenu en solution dans 100 ml de DMF, ajoute 40 ml d'iodure de méthyle et chauffe à 80°C pendant 3 heures. On évapore sous vide le mélange réactionnel, reprend le résidu au DCM, lave par une solution saturée d'hydrogénocarbonate de sodium, sèche sur sulfate de magnésium et évapore sous vide le solvant. On chromatographie sur silice en éluant par du toluène. On distille sous vide et obtient 13 g du produit attendu, T_{Eb} = 138°C sous 0,02 bar de pression.
D) 2-Chloro-5-méthoxytoluène.
   A une solution de 20 g de 4-chloro-3-méthylphénol dans 200 ml d'EtOH on ajoute 42,05 g d'une solution à 50 % en poids d'hydroxyde de césium dans l'eau. On évapore sous vide, reprend le résidu dans l'isopropanol, évapore à nouveau sous vide et répète trois fois cette opération. On met le sel de césium ainsi obtenu en solution dans 100 ml de DMF, ajoute 30 ml d'iodure de méthyle et chauffe à 80°C pendant 3 heures. On évapore sous vide le mélange réactionnel, reprend le résidu au DCM, lave à l'eau, par une solution saturée de carbonate de sodium, sèche sur sulfate de magnésium et évapore sous vide le solvant. On chromatographie sur silice en éluant par du toluène. On distille sous vide et obtient 14 g du produit attendu, T_{Eb} = 105°C sous 0,02 bar de pression.
E) 2,5-Diméthoxytoluène.

On chauffe à reflux pendant 4 jours un mélange de 12 g de méthylhydroquinone, 45g de carbonate de potassium, 45 g de diméthylsulfate dans 300 ml d'acétone anhydre. Après refroidissement, on filtre le mélange réactionnel et évapore sous vide le filtrat. On reprend le résidu dans 150 ml d'ammoniaque concentrée, laisse 2 heures sous agitation, dilue à l'eau, extrait au DCM, sèche sur sulfate de magnésium et évapore sous vide le solvant. On chromatographie le résidu sur silice H en éluant par le mélange Heptane/DCM (50/50 ; v/v). On obtient 12 g du produit attendu.

Spectre de RMN à 200 MHz dans DMSO.
2,05 ppm : s : 3H
3,60 ppm : s : 3H
3,65 ppm : s : 3H
6,5 à 6,9 ppm : m : 3H

### PREPARATION II - Alphachlorocétones de formule (V).

A) 1-(2,6-Diméthoxy-4-méthylphényl)-2-chloro-1-éthanone.
   7,61 g de 3,5-diméthoxytoluène et 6,10 g de tétraméthyléthylènediamine sont mis en solution, sous azote, dans 150 ml d'hexane. La solution est refroidie à 0°C, on ajoute 32,8 ml de butyllithium 1,6 M dans l'hexane, agite le mélange à 10°C pendant 20 minutes puis à 20°C pendant 1 heure. Au dérivé lithié refroidi à -10°C, on ajoute en 20 minutes une solution refroidie à 0°C de 6,13 g de N-méthoxy-N-méthylchloroacétamide dans 50 ml de THF. Le mélange réactionnel est laissé une heure à une température comprise entre 0 et 5°C, une heure à 20°C, puis versé dans 100 ml d'eau. On extrait avec deux fois 300 ml d'éther diéthylique, lave les phases éthérées avec une solution saturée de chlorure de sodium, sèche les phases organiques sur sulfate de magnésium, filtre et concentre sous vide. Le résidu est chromatographié sur silice, en éluant par le mélange DCM/hexane (50/50 ; v/v). La concentration des fractions pures fournit 1,6 g du produit attendu ; F = 82-84°C.
B) 1-(2,4,6-Triméthoxy-3-pyridinyl)-2-chloro-1-éthanone (selon Chem. Pharm. Bull., 1986, *34*, 3658 et J. Am. Chem. Soc., 1932, *54*, 727).
   24 g de 2,6-dichloropyridine, 200 ml d'acide trifluoroacétique et 28 ml d'eau oxygénée à 33 % sont chauffés à 100°C pendant 4 heures. On refroidit puis ajoute 600 ml d'eau et concentre sous vide jusqu'à un volume de 50-100 ml. On alcalinise avec de l'hydrogénocarbonate de sodium puis extrait au DCM, décante la phase organique et sèche sur sulfate de sodium. On filtre, concentre sous vide et recristallise le résidu de l'AcOEt pour obtenir 18,8 g de N-oxyde de 2,6-dichloropyridine ; F = 138-140°C.
   18,8 g du composé préparé précédemment sont chauffés à reflux pendant 6 heures dans 40 ml d'oxychlorure de phosphore et abandonnés une nuit à TA puis on concentre le mélange sous vide. Le résidu est versé dans l'eau froide puis successivement on neutralise avec du carbonate de sodium, extrait à l'éther, décante la phase éthérée, la sèche sur sulfate de sodium, filtre et concentre sous vide. Le résidu est chromatographié sur gel de silice, en éluant par le mélange DCM/heptane (60/40 ; v/v). La concentration des fractions pures fournit 14,6 g de 2,4,6-trichloropyridine.
   Un mélange de 14,6 g du produit préparé précédemment et de 129,7 g de méthylate de sodium dans 400 ml de MeOH est chauffé à reflux pendant une nuit. On ajoute 0,7 litre d'eau puis successivement, extrait au DCM, lave l'extrait organique à l'eau et sèche sur sulfate de sodium. On concentre sous vide et le résidu est recristallisé du pentane pour fournir 9,5 g de 2,4,6-triméthoxypyridine; F = 47-49°C.
   On additionne sous azote à 15 ml de THF anhydre à -40°C, 7,5 ml de méthyllithium 1,6 M dans l'éther, 0,02 ml de diisopropylamine puis on agite pendant 5 minutes et additionne à -40°C, en 10 minutes, 1,13 g du dérivé de la pyridine préparé précédemment en solution dans 10 ml de THF. On agite le mélange pendant 3 heures à 0°C. On refroidit ensuite à -70°C et ajoute en 5 minutes 0,824 g de N-méthyl-N-méthoxychloroacétamide en solution dans 20 ml de THF et laisse remonter la température jusqu'à 10°C en une heure. On verse le mélange réactionnel dans 300 ml d'eau froide saturée en chlorure de sodium et extrait à l'éther. L'extrait organique est successivement lavé avec une solution saturée en chlorure de sodium, décanté, séché sur sulfate de sodium, filtré et concentré sous vide. Le résidu est chromatographié sur gel de silice, en éluant par le mélange cyclohexane/AcOEt (80/20 ; v/v). La concentration des fractions de produit pur fournit 0,61 g de l'éthanone attendue ; F = 85-87°C.
C) 1-(2,4-Diméthoxy-5-méthylphényl)-2-chloro-1-éthanone.
   On prépare ce composé selon Chem. Pharm. Bull., 1991, 39 (9), 2400-2407.
   On refroidit à 0°C une suspension de 5,24 g d'AlCl₃, 0,52 g de ZnCl₂ dans 40 ml de 1,2-dichloroéthane et ajoute, goutte à goutte, une solution de 5,0 g de 2,4-diméthoxytoluène dans 20 ml de 1,2-dichlorcéthane. On refroidit ensuite à -10°C et ajoute, goutte à goutte, une solution de 2,9 ml de chlorure de chloracétyle dans 1,5 ml de 1,2-dichlorcéthane, en maintenant la température du milieu réactionnel entre -10°C et -7°C. On laisse sous agitation en laissant remonter la température à TA, verse le milieu réactionnel sur un mélange de glace et d'HCl concentré, extrait au DCM, lave les phases organiques jointes à l'eau, sèche sur sulfate de magnésium et évapore sous vide les solvants. On reprend le résidu dans l'heptane et filtre le précipité formé. On obtient 3,0 g du produit attendu, F = 166-167°C.
D) 1-(4-trifluorométhyl-2,6-diméthoxyphényl)-2-chloro-1-éthanone.

On refroidit à 10°C une solution de 9,73 g de 3-amino-5-méthoxy-1-trifluorométhylbenzène dans 400 ml d'HCl 2N et ajoute en 10 minutes une solution de 3,80 g de nitrite de sodium dans 20 ml d'eau. On laisse 30 minutes sous agitation à 10°C et ajoute une solution de 800 ml d'H₂SO₄ concentré dans 800 ml d'eau en maintenant la température en dessous de 20°C. On chauffe ensuite à 95°C pendant 2 heures et abandonne une nuit à TA. On ajoute 1000 g de glace au milieu réactionnel, extrait à l'éther, lave par une solution saturée de chlorure de sodium, sèche sur sulfate de sodium et évapore sous vide le solvant. On obtient 9,8 g de 3-hydroxy-5-méthoxy-1-trifluorométhylbenzène, F = 75°C (selon J. Chem. Soc., 1951, 2013).

A une solution de 9,8 g du composé préparé précédemment dans 100 ml d'acétone, on ajoute 7,90 g de K₂CO₃ et chauffe à 50°C. On ajoute ensuite, goutte à goutte, à cette température et en 20 minutes, 6,74 g de diméthylsulfate et chauffe à reflux pendant 2 heures. On évapore sous vide le mélange réactionnel, reprend le résidu par 30 ml d'une solution d'ammoniaque à 20 % et par 50 ml d'eau, extrait à l'éther, lave par une solution saturée de chlorure de sodium, sèche sur sulfate de sodium et évapore sous vide le solvant. On obtient 8,7 g de 3,5-diméthoxy-1-trifluorométhylbenzène après distillation sous vide, T_{Eb} = 92-94°C sous 0,02 bar de pression.

A une solution de 8,6 g du composé préparé précédemment dans 100 ml d'hexane, on ajoute 5,09 g de tétraméthyléthylènediamine. On refroidit à -5°C et ajoute, sous atmosphère d'azote, en 15 minutes, 27,4 ml d'une solution 1,6 M de butyllithium dans l'hexane puis laisse sous agitation pendant 1 heure et 30 minutes à une température comprise entre -5°C et +5°C. On ajoute ensuite la solution de dérivé lithié à une solution, refroidie à -25°C, de 5,41 g de N-méthoxy-N-méthylchloroacétamide dans 45 ml de THF et laisse 2 heures sous agitation en laissant remonter la température à +5°C. On ajoute 100 ml d'eau ; extrait à l'éther, lave par une solution saturée de chlorure de sodium, sèche sur sulfate de sodium et évapore sous vide le solvant. On obtient 3,6 g du produit attendu après cristallisation dans l'hexane, F = 120-122°C.

Les cétones chlorées de formule (V) décrites dans le TABLEAU I ont été préparées selon un des procédés mis en oeuvre précédemment et en utilisant les produits de départ appropriés.

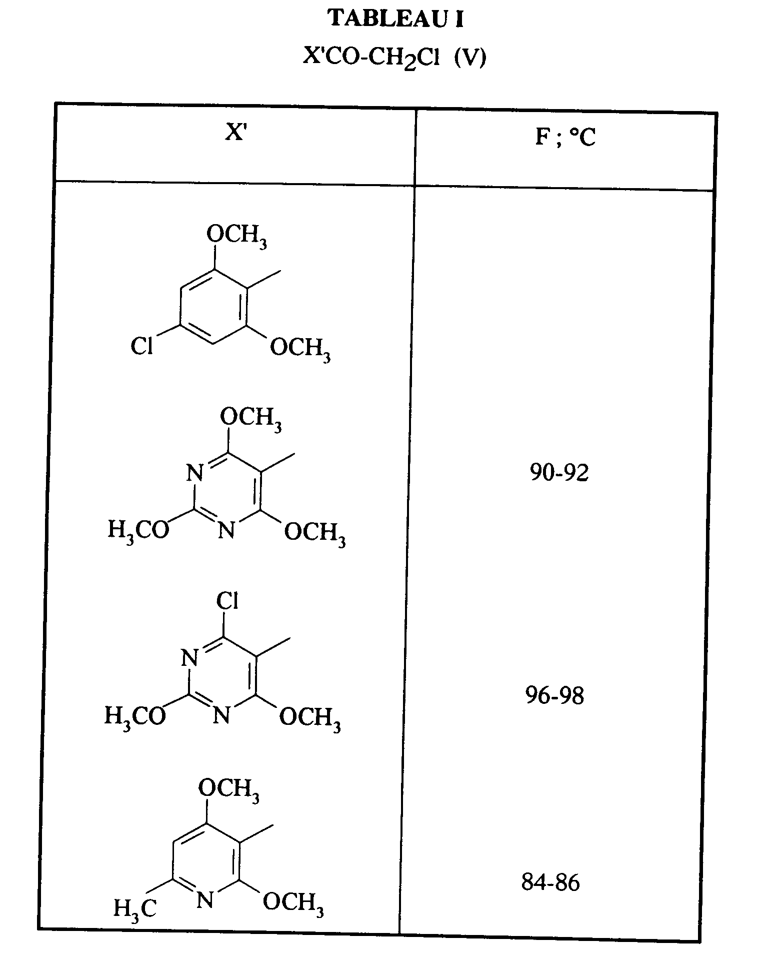

### PREPARATION III. Cétones aromatiques de formule (VII).

A) 1-(5-Chloro-2,4-diméthoxyphényl)-1-éthanone.
   On refroidit à 0°C un mélange de 2 g de 1-chloro-2,4-diméthoxybenzène, 0,9 g de chlorure d'acétyle dans 20 ml de CCl₄ et ajoute goutte à goutte une solution de 1,3 ml de TiCl₄ dans 7 ml de CCl₄. On laisse 2 heures sous agitation en laissant remonter la température à TA. On verse le mélange réactionnel sur un mélange d'HCl concentré et de glace, extrait au DCM, sèche sur sulfate de magnésium et évapore sous vide les solvants. On chromatographie sur silice H en éluant par le mélange DCM/heptane (70/30 ; v/v). On obtient 1,19 g du produit attendu, F = 138°C.
B) 1-(5-Chloro-2,4-diméthoxyphényl)-1-propanone.
   On refroidit à 0°C un mélange de 2,01 g de 1-chloro-2,4-diméthoxybenzène, 1,08 g de chlorure de propionyle dans 20 ml de CCl₄ et ajoute goutte à goutte une solution de 1,3 ml de TiCl₄ dans 7 ml de CCl₄. On laisse 2 heures sous agitation en laissant remonter la température à TA. On verse le mélange réactionnel sur un mélange d'HCl concentré et de glace, extrait au DCM, sèche sur sulfate de magnésium et évapore sous vide les solvants. On chromatographie sur silice H en éluant par le mélange DCM/heptane (80/20 ; v/v). On obtient 1,14 g de produit attendu, F = 115°C.
C) 1-(5-Chloro-2-méthoxy-4-méthylphényl)-1-éthanone.
   On refroidit à 0°C, sous atmosphère d'azote, une suspension de 2,12 g d'AlCl₃ dans 20 ml de CCl₄ et ajoute, goutte à goutte, une solution de 1,25 g de chlorure d'acétyle dans 10 ml de CCl₄. On ajoute ensuite, goutte à goutte, une solution de 2,5 g de 2-chloro-5-méthoxytoluène dans 10 ml de CCl₄ et laisse 2 heures sous agitation en laissant remonter la température à TA. On verse sur un mélange d'HCl concentré et de glace, extrait au DCM, sèche sur sulfate de magnésium et évapore sous vide le solvant. On chromatographie sur silice H en éluant par le mélange DCM/heptane (70/30 ; v/v). On obtient 0,68 g du produit attendu, F = 83°C.
D) 1-(5-Chloro-2-méthoxy-4-méthylphényl)-1-propanone.
   On refroidit à 0°C, sous atmosphère d'azote, une suspension de 2,55 g d'AlCl₃ dans 30 ml de DCM et ajoute, goutte à goutte, une solution de 1,77 g de chlorure de propionyle dans 15 ml de DCM. On ajoute ensuite, goutte à goutte, une solution de 3 g de 2-chloro-5-méthoxytoluène dans 15 ml de DCM et laisse 2 heures sous agitation. On verse le mélange réactionnel sur un mélange d'HCl concentré et de glace, extrait au DCM, sèche sur sulfate de magnésium et évapore sous vide le solvant. On chromatographie sur silice en éluant par le mélange DCM/heptane (70/30 ; v/v). On obtient 2,2 g du produit attendu, F = 79°C.
E) 1-(5-Ethyl-2,4-diméthoxyphényl)-1-propanone.
   On refroidit à +4°C une suspension de 10 g de 4-éthylrésorcinol dans 20 ml d'éthérate de trifluorure de bore et ajoute, goutte à goutte, 11,7 g d'anhydride propionique. On chauffe à 75°C pendant 6 heures et verse, après refroidissement, le mélange réactionnel sur un mélange d'eau et de glace. On laisse 2 heures sous agitation, filtre le précipité formé, lave ce dernier à l'eau, le reprend dans AcOEt, lave la phase organique à l'eau, sèche sur sulfate de sodium et évapore sous vide le solvant. On chromatographie sur silice en éluant par du DCM puis par un mélange DCM/AcOEt (90/10 ; v/v). On obtient 9,32 g de 1-(5-éthyl-2,4-dihydroxyphényl)-1-propanone, F = 74-75°C.
   On chauffe à reflux pendant 48 heures une suspension de 5 g du composé préparé précédemment, 30 g de carbonate de potassium, 30 ml de diméthylsulfate dans 500 ml d'acétone. Après refroidissement, on filtre un insoluble, évapore sous vide le filtrat et reprend le résidu dans 100 ml d'ammoniaque concentrée. Après 1 heure d'agitation, on ajoute 400 ml d'eau, filtre le précipité formé, lave ce dernier à l'eau, le reprend dans du DCM, lave la phase organique à l'eau, sèche sur sulfate de magnésium et évapore sous vide le solvant. On obtient 5,68 g du produit attendu, F = 64-65°C.
F) 1-(2,4-Diméthoxyphényl)-3-phényl-1-propanone.

A une suspension de 43,2 g d'AlCl₃, 37,5 g de 1,3-diméthoxybenzène dans 210 ml de CCl₄ on ajoute, goutte à goutte, une solution de 45,5 g de chlorure de l'acide 3-phénylpropanoïque dans 50 ml de CCl₄. On laisse 1 heure sous agitation à TA et verse le milieu réactionnel sur un mélange de 400 g de glace et 150 ml d'HCl concentré. Après 30 minutes d'agitation, on extrait au DCM, lave les phases organiques jointes par une solution saturée d'hydrogénocarbonate de sodium, sèche sur sulfate de sodium et évapore sous vide les solvants. On obtient 66,5 g d'huile du produit attendu qui est utilisé tel quel.

Les cétones aromatiques de formule (VII) décrites dans le TABLEAU II se préparent selon un des procédés mis en oeuvre précédemment et en utilisant les produits de départ appropriés.

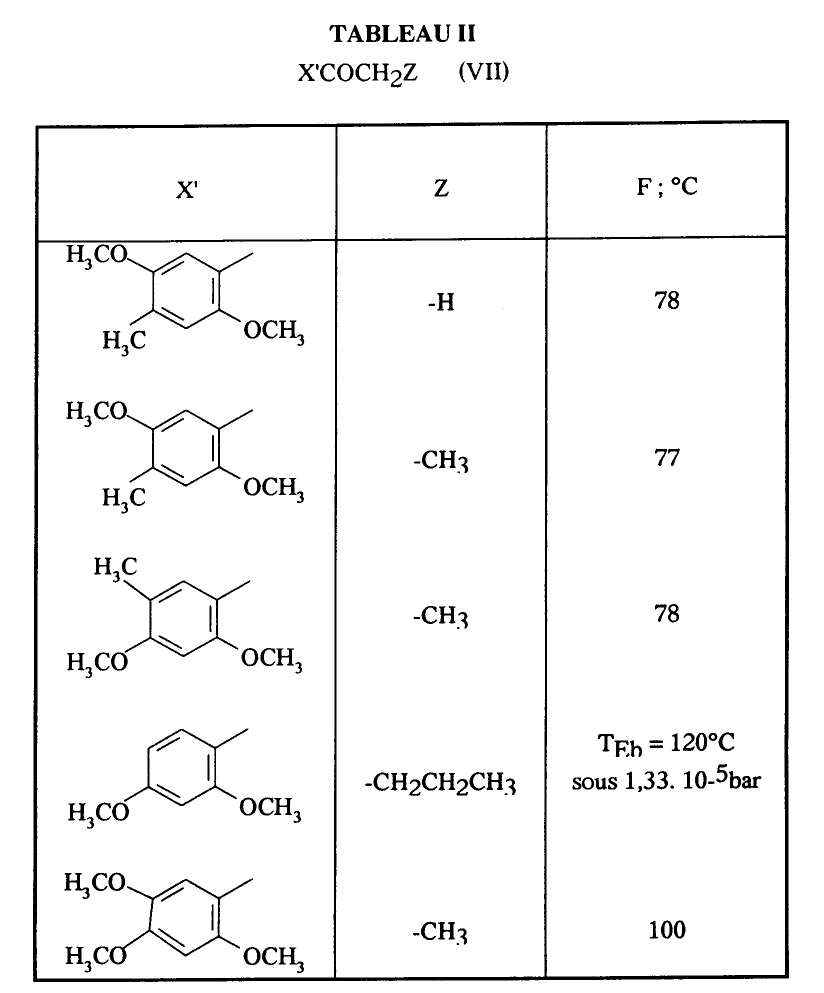

### PREPARATION IV: 2-Aminothiazoles de formule (III).

A) 2-Amino-4-(2,6-diméthoxy-4-méthylphényl)thiazole.
   0,41 g du produit préparé précédemment selon la PREPARATION II. A et 0,164 g de thiourée sont mis en solution dans 50 ml d'EtOH absolu. Le mélange réactionnel est chauffé à reflux pendant 18 heures puis concentré sous vide. Le résidu est repris dans 100 ml d'une solution de NaOH 2N, puis on extrait avec deux fois 200 ml de DCM, décante les phases organiques, sèche sur sulfate de sodium, filtre et concentre sous vide. Le résidu cristallise dans l'éther pour fournir 0,34 g de l'aminothiazole attendu ; F 204-206°C.
B) 2-Amino-4-(2,4,6-triméthoxy-3-pyridyl)thiazole.
   On chauffe à reflux pendant 24 heures, un mélange de 0,55 g de cétone obtenue selon la PREPARATION II. B et de 0,21 g de thiourée dans 25 ml d'EtOH absolu. On concentre le mélange réactionnel sous vide, reprend la résidu dans l'eau et ajoute une solution de carbonate de sodium à 10 %. On extrait à l'AcOEt, sèche la phase organique sur sulfate de sodium, filtre et concentre sous vide. Le résidu cristallise dans un minimum d'éther iso. On obtient 0,51 g du thiazole attendu ; F = 191°C.
C) 2-Amino-4-(5-chloro-2,4-diméthoxyphényl)thiazole.
   A une solution de 1,08 g du composé obtenu à la PREPARATION III A dans 20 ml de CCl₄, on ajoute, goutte à goutte, à TA une solution de 0,26 ml de brome dans 10 ml de CCl₄. On lave la phase organique à l'eau, sèche sur sulfate de magnésium et évapore sous vide le solvant. On reprend le résidu dans 20 ml d'EtOH, ajoute 2 g de thiourée et chauffe à reflux pendant 3 heures. On évapore sous vide, extrait au DCM, lave par une solution saturée de carbonate de sodium, sèche sur sulfate de magnésium et évapore sous vide. On chromatographie sur silice en éluant par le mélange DCM/MeOH (100/1 ; v/v). On obtient 0,92 g du produit attendu, F = 162°C.
D) 2-Amino-4-(5-chloro-2,4-diméthoxyphényl)-5-méthylthiazole.

A une solution de 1,12 g du composé obtenu à la PREPARATION III B dans 20 ml de DCM, on ajoute, goutte à goutte, à TA une solution de 0,25 ml de brome dans 5 ml de DCM. On lave la phase organique à l'eau, sèche sur sulfate de magnésium et évapore sous vide le solvant. On reprend le résidu dans 20 ml d'EtOH, ajoute 1,0 g de thiourée et chauffe à reflux pendant 2 heures. On évapore sous vide, reprend le résidu par une solution saturée de carbonate de sodium, extrait au DCM, sèche sur sulfate de magnésium et évapore sous vide. On reprend le résidu dans l'éther et filtre le précipité formé. On obtient 1,26 g du produit attendu, F = 188°C.

Les 2-aminothiazoles de formule (III) décrits dans le TABLEAU III ci-après ont été synthétisés en appliquant les procédés précédents.

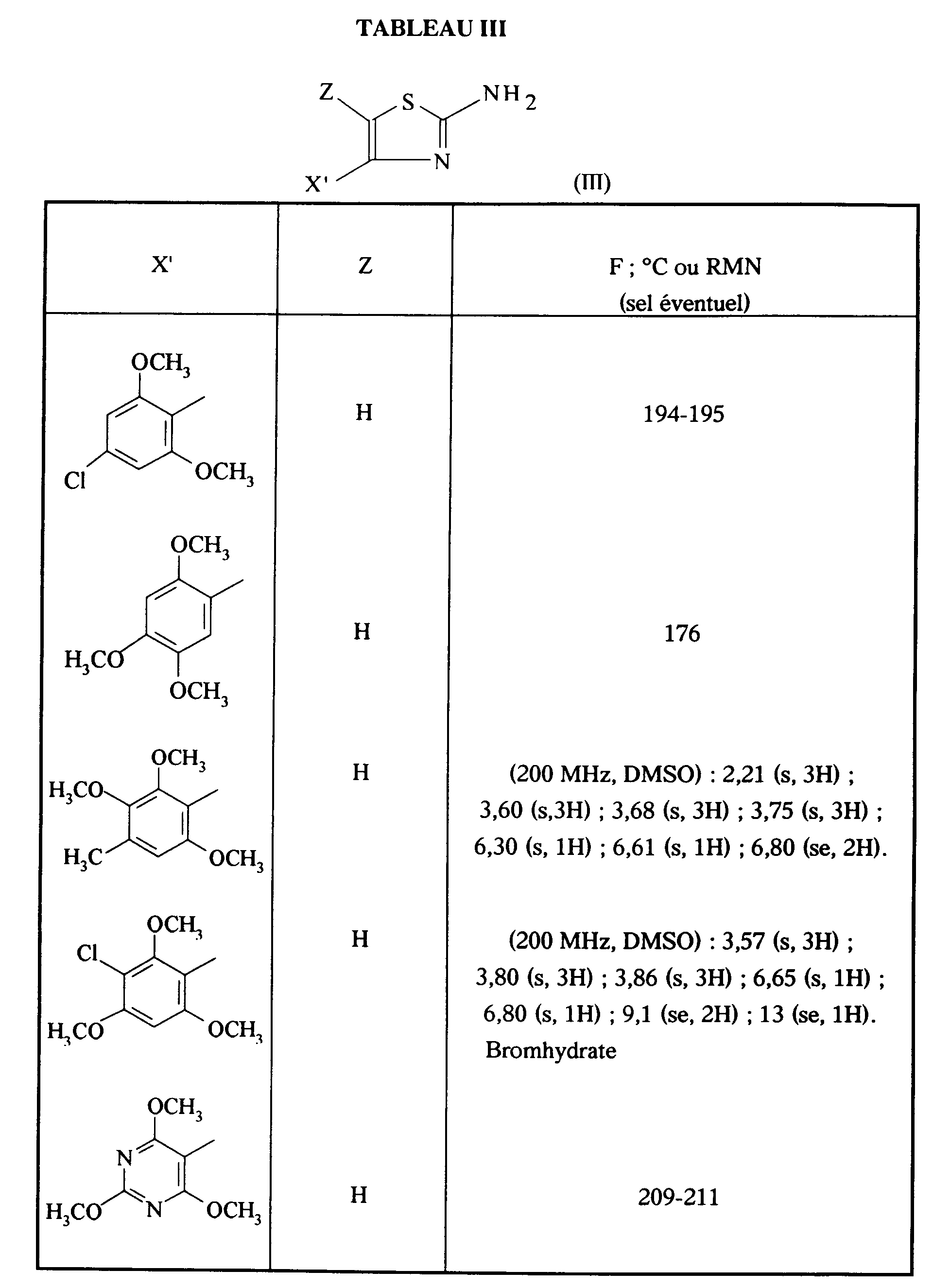

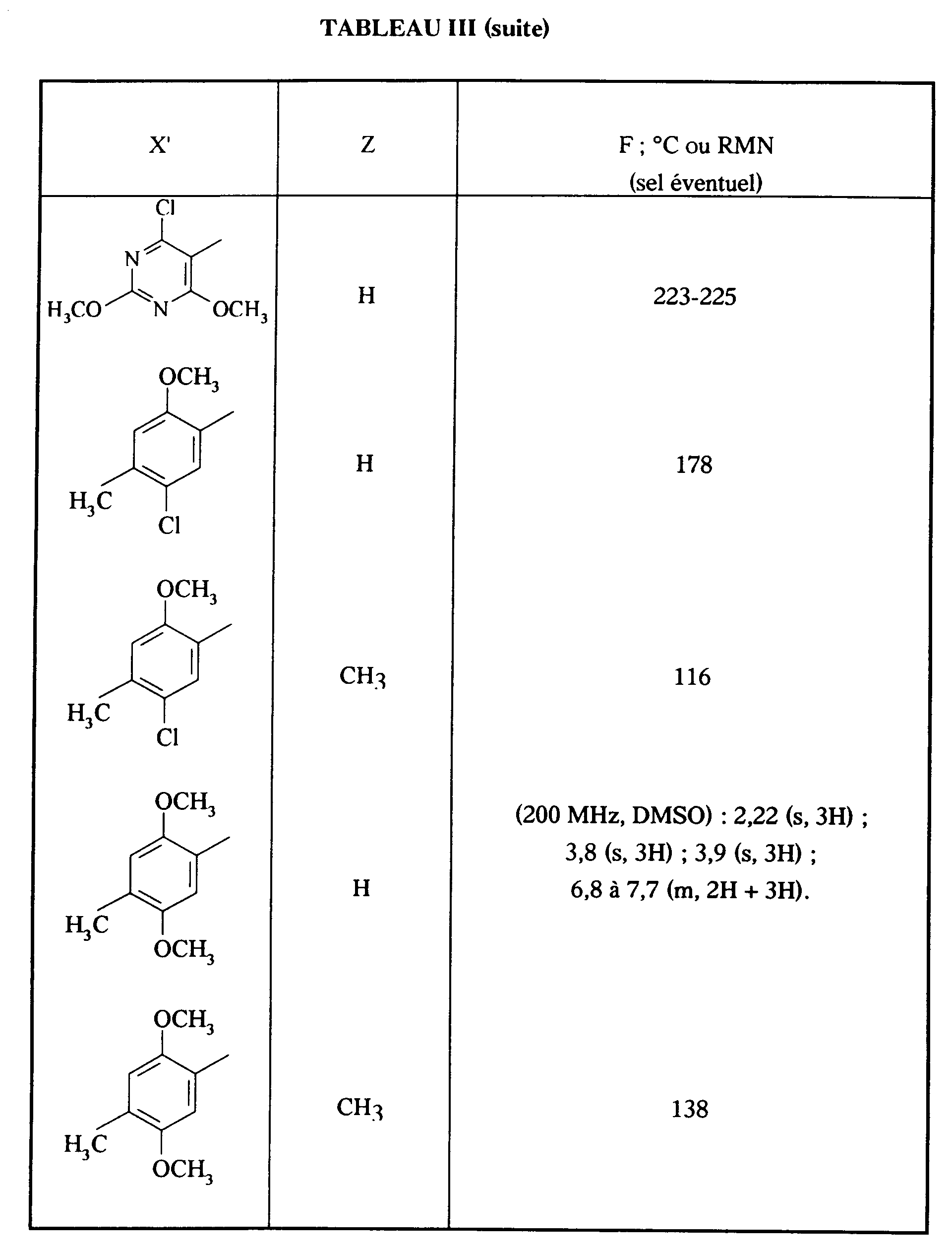

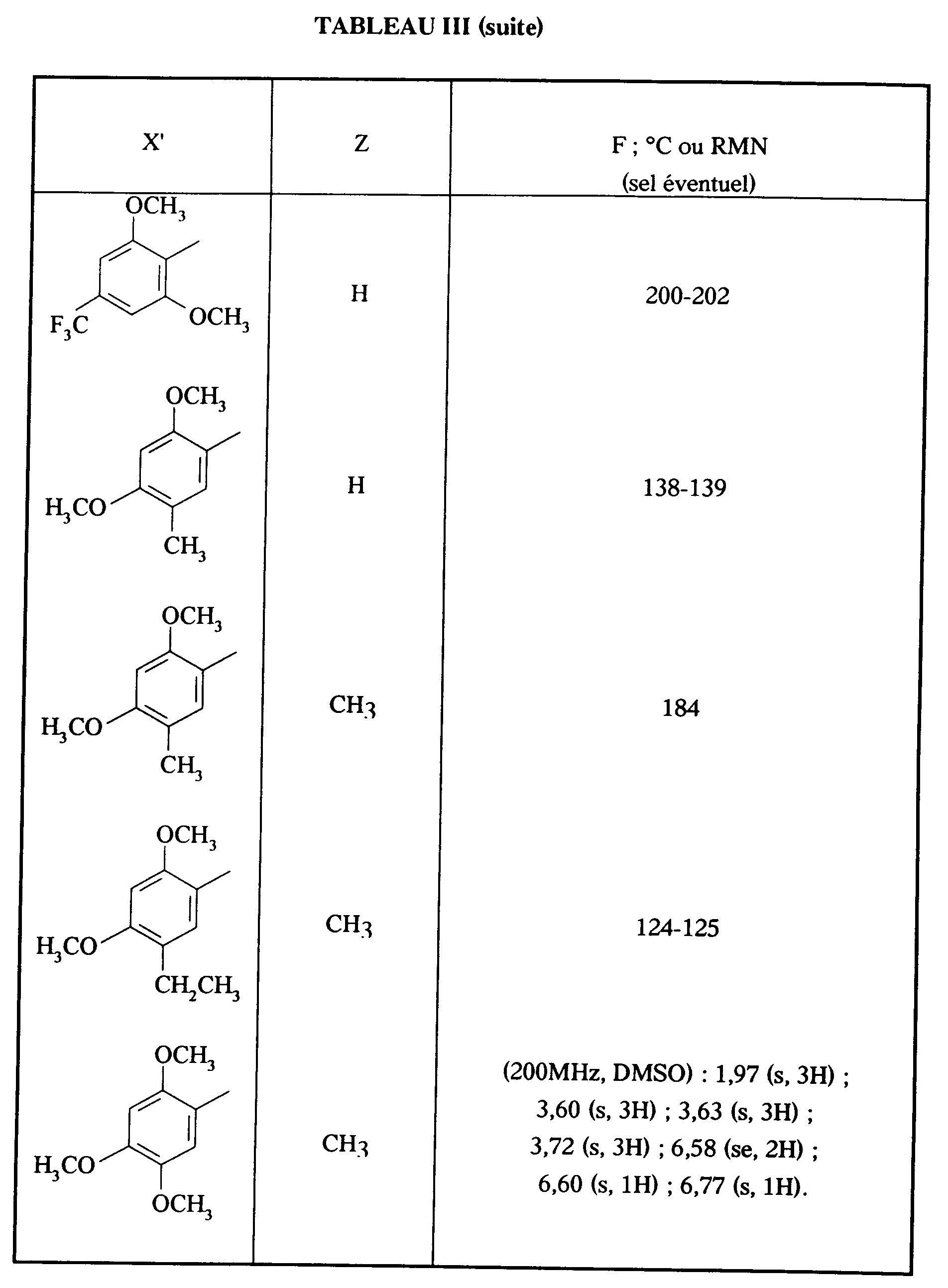

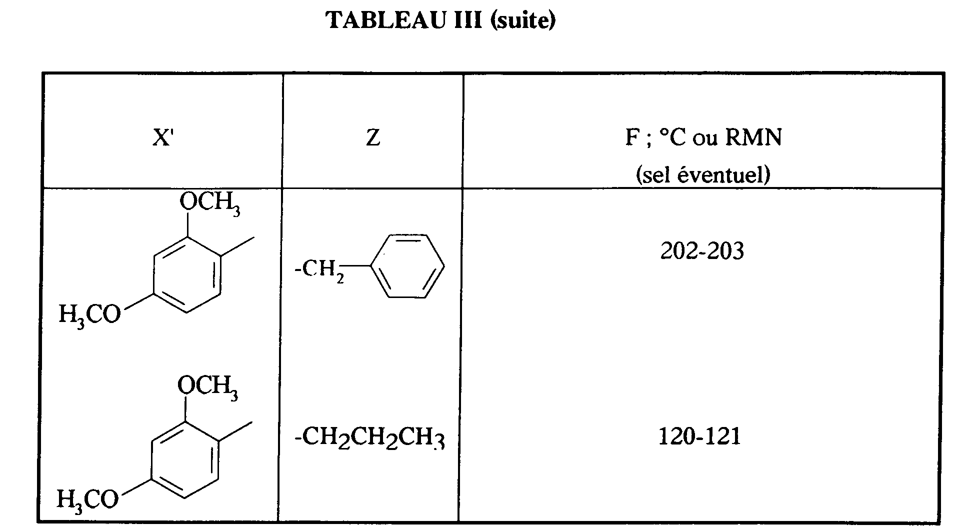

### PREPARATION V: Acides indolecarboxyliques (II).

Les acides indolecarboxyliques sont préparés selon EP-A-0432040.

### EXEMPLE 1

Chlorhydrate de N-[4-(2,6-diméthoxy-4-méthylphényl)thiazol-2-yl]-1*H*-indole-2-carboxamide monohydraté. (méthode A). 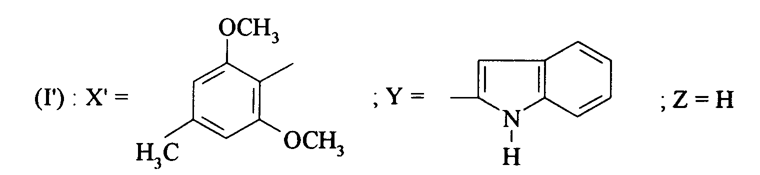
0,33 g de l'amine obtenue précédemment selon la PREPARATION IV. A, 0,29 g d'acide N-acétylindole-2-carboxylique, 0,7 g de BOP et 0,16 g de triéthylamine sont mis en solution dans 40 ml de DCM. Le mélange réactionnel est agité pendant 48 heures à TA, successivement on ajoute 50 ml d'une solution tampon pH = 2, décante la phase organique, sèche sur sulfate de sodium, filtre et concentre sous vide. Le résidu est repris dans 80 ml d'EtOH 96°, on ajoute 10 ml d'une solution NaOH 2N et agite le mélange réactionnel à TA pendant 2 heures et demie. La solution est neutralisée avec 1,8 ml d'HCl concentré. Le précipité formé est séparé par filtration, lavé à l'eau et séché sous vide à 60°C pour fournir 0,45 g du composé attendu, F = 250-252°C.

### EXEMPLE 2

N-[4-(2,4,6-Triméthoxy-3-pyridinyl)thiazol-2-yl]-1*H*-indole-2-carboxamide. (méthode A).

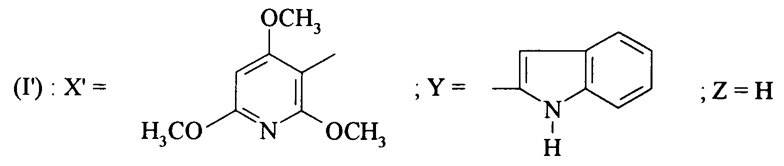

On agite pendant 24 heures, à TA, une solution de 25 ml de DCM. 0,5 g d'aminothiazole obtenu selon la PREPARATION IV. B, 0,40 g d'acide N-acétylindole-2-carboxylique, 0,99 g de BOP et 0,23 g de triéthylamine. On ajoute 20 ml d'eau, décante la phase organique, sèche sur sulfate de sodium, filtre et concentre sous vide. Le résidu est chromatographié sur gel de silice H, éluant DCM/MeOH (100/1 ; v/v). On élimine une impureté de tête puis on élue le produit de couplage correspondant au dérivé acétylé sur l'azote indolique. Ces fractions sont concentrées sous vide et le résidu est dissous dans 50 ml d'EtOH absolu. On ajoute à cette solution 5 ml d'une solution de NaOH 2N et agite le mélange réactionnel à TA pendant 1 H 30. On neutralise par addition de 0,85 ml d'HCl concentré et concentre sous vide. Le résidu est repris dans l'eau additionnée de carbonate de sodium, on filtre et lave successivement le précipité à l'eau puis à l'EtOH absolu pour obtenir 0,44 g du composé attendu ; F = 285-287°C.

### EXEMPLE 3

N-[4-(2,6-Diméthoxy-4-méthylphényl)thiazol-2-yl-]quinoléine-3-carboxamide.
(méthode B).

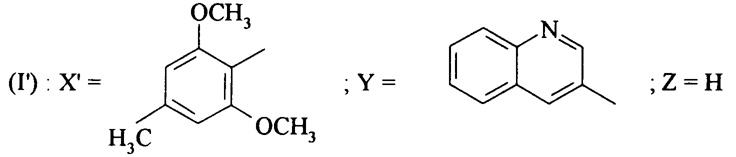

1 g de 2-amino-4-(2,6-diméthoxy-4-méthylphényl)thiazole, 0,76 g d'acide quinoléine-3-carboxylique, 0,65 ml de triéthylamine et 2,15 g de BOP sont mis en solution dans 10 ml de DMF et on abandonne le mélange réactionnel à TA pendant 48 heures. On verse alors celui-ci sur une solution tampon pH = 2, un précipité est séparé par filtration et le solide jaune est successivement lavé à l'eau, agité dans une solution de carbonate de sodium à 5 %, filtré puis dissous dans le DCM. On lave avec une solution de carbonate de sodium à 5 %, puis successivement, décante la phase organique, sèche sur sulfate de magnésium, filtre et concentre sous vide. Le résidu est agité dans l'éther, filtré et séché pour fournir 1,58 g du composé attendu ; F = 245-246°C.

### EXEMPLE 4

N-[4-(4-Chloro-2,6-Diméthoxyphényl)thiazol-2-yl]-1-(carboxyméthyl)indole-2-carboxamide. (méthode C).

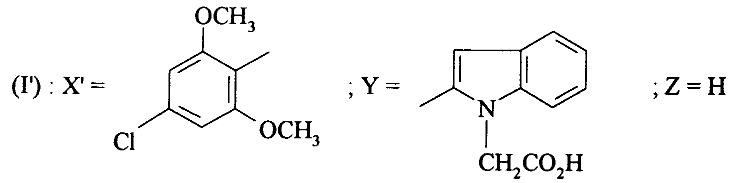

On met en solution dans 5 ml de DMF, 0,7 g de 2-amino-4-(2,6-diméthoxy-4-chlorophényl)thiazole, 0,61 g d'acide N-(méthoxycarbonylméthyl)indole-2-carboxylique, 0,42 ml de triéthylamine et 1,4 g de BOP puis on abandonne le mélange réactionnel pendant 48 heures à TA. On verse le mélange sur du tampon sulfate pH = 2 puis on filtre le précipité qui est ensuite lavé à l'eau et dissous dans DCM. On lave la solution avec une solution d'hydrogénocarbonate de sodium 5 % puis avec du tampon sulfate pH = 2, décante la phase organique, sèche sur sulfate de sodium, filtre et concentre sous vide. Le résidu est chromatographié sur gel de silice H. La concentration des fractions de produit pur fournit 1,08 g de l'ester méthylique attendu ; F = 236-237°C.

1,08 g de l'ester méthylique préparé ci-dessus sont mis en solution dans 100 ml d'EtOH 95° en présence de 1,5 ml de NaOH 2N. On agite le mélange réactionnel à TA pendant 48 heures et concentre sous vide. Le résidu est repris dans l'eau puis on ajoute goutte à goutte de l'HCl concentré jusqu'à pH = 1. On filtre le précipité et le sèche pour obtenir 0,84 g du chlorhydrate attendu ; F > 300°C.

### EXEMPLE 5

Trifluoroacétate de N-[4-(2,6-diméthoxy-4-méthylphényl)thiazol-2-yl]-1-(carboxy-méthyl)indole-2-carboxamide. (méthode D).

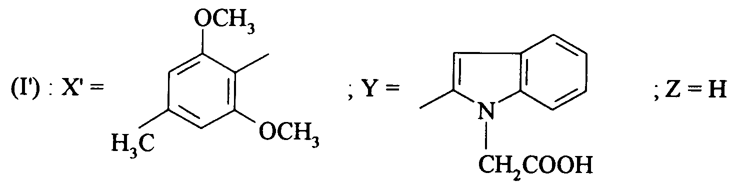

1,07 g de N-[4-(2,6-diméthoxy-4-méthylphényl)-2-thiazolyl]-1-(*tert*-butoxy-carbonylméthyl)indole-2-carboxamide (préparé selon EP-A-0432040) sont mis en solution dans un mélange de 2 ml d'anisole et 20 ml de TFA. Le mélange réactionnel est abandonné pendant 3/4 d'heure à TA puis concentré sous vide. Le résidu est repris dans l'éther puis on filtre le précipité et sèche à l'étuve pour obtenir 1,13 g du composé attendu ; F = 223-224°C.

### EXEMPLE 6

N-[4-(2,3,6-triméthoxy-4-méthylphényl)thiazol-2-yl]-1*H*-indole-2-carboxamide (méthode E).

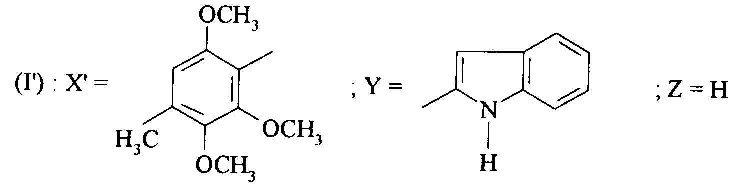

0,16 g de 2-amino-4-(2,3,6-triméthoxy-4-méthylphényl)thiazole sont dissous dans 10 ml de DMF. On ajoute 0,18 g de l'acide N-(*ter*t-butyloxycarbonylméthyl)indoline-2-carboxylique, 0,2 ml de triéthylamine, 0,38 g de BOP et laisse le mélange réactionnel sous agitation pendant 48 heures. On ajoute 100 ml d'eau, extrait à l'AcOEt, décante la phase organique, sèche sur sulfate de sodium et concentre sous vide. Le résidu est dissous dans 10 ml de CHCl₃ puis on ajoute 10 ml de TFA et agite le mélange réactionnel à TA pendant 2 heures 30. On concentre sous vide en ajoutant trois fois 20 ml de benzène. Le résidu est dissous dans 20 ml de diméthoxyéthane puis on ajoute 0,1 ml de triéthylamine et 0,112 g de DDQ et laisse le mélange réactionnel pendant une nuit à température ambiante. On concentre sous vide, reprend le résidu dans l'AcOEt et lave successivement avec une solution de NaOH 1N, avec une solution de KHSO₄, avec une solution de chlorure de sodium ; on décante la phase organique, sèche sur sulfate de sodium, filtre et concentre sous vide. Le résidu est chromatographié sur gel de silice, éluant CHCl₃/AcOEt (50/50 ; v/v). Les fractions de produits purs sont concentrées sous vide et le résidu est concrétisé dans le pentane pour fournir 0,08 g du produit attendu ; F = 200°C.

### EXEMPLE 7

N-[4-(5-chloro-2,4-diméthoxyphényl)thiazol-2-yl]-1H-indole-2-carboxamide
(méthode F).

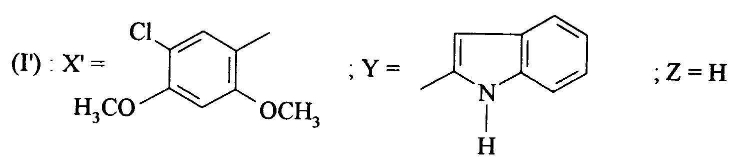

On agite à TA pendant une nuit un mélange de 0,9 g du composé obtenu à la PREPARATION IV.C, 0,67 g d'acide N-acétylindole-2-carboxylique, 1,5 g de BOP, 0,46 ml de triéthylamine dans 4 ml de DMF. On verse le mélange réactionnel sur une solution tampon pH = 2, filtre le précipité formé et lave ce dernier à l'eau. On reprend le précipité au DCM, lave par une solution saturée d'hydrogénocarbonate de sodium, sèche sur sulfate de magnésium et évapore sous vide. On chromatographie sur silice en éluant par le mélange DCM/AcOEt (100/1 ; v/v). On reprend le dérivé acétylé sur l'azote indolique obtenu dans 30 ml d'EtOH, ajoute 1 g de carbonate de sodium et agite une nuit à TA. On évapore sous vide le mélange réactionnel, reprend le résidu dans l'eau, filtre le précipité formé, lave à l'eau et sèche sous vide à l'étuve. On obtient 0,56 g du produit attendu, F = 293°C.

### EXEMPLE 8

N-[4-(5-chloro-2,4-diméthoxyphényl)-5-méthylthiazol-2-yl]-1H-indole-2-carboxamide (méthode F).

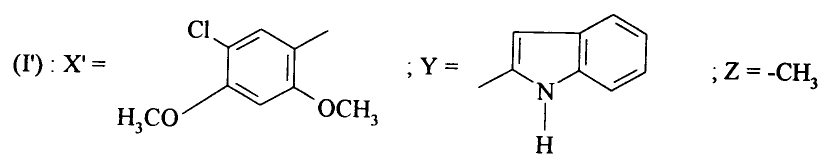

On agite à TA pendant une nuit, un mélange de 1,24 g du composé obtenu à la PREPARATION IV. D, 0,88 g d'acide N-acétylindole-2-carboxylique, 1,95 g de BOP, 0,60 ml de triéthylamine dans 4 ml de DMF. On verse le mélange réactionnel sur une solution tampon pH = 2, filtre le précipité formé et lave ce dernier à l'eau. On reprend le précipité au DCM, lave par une solution saturée d'hydrogénocarbonate de sodium, sèche sur sulfate de magnésium et évapore sous vide. On chromatographie sur silice en éluant par le mélange DCM/AcOEt (100/1 ; v/v). On reprend le dérivé acétylé sur l'azote indolique obtenu dans 30 ml d'EtOH, ajoute 2 g de carbonate de sodium et agite une nuit à TA. On évapore sous vide le mélange réactionnel, reprend le résidu à l'eau, filtre le précipité formé, lave à l'eau, puis à l'éther et sèche sous vide à l'étuve. On obtient 0,81 g du produit attendu, F = 249°C.

### EXEMPLE 9

Trifluoroacétate de N-[4-(5-chloro-2,4-diméthoxyphényl)-5-méthylthiazol-2-yl]-1-(carboxyméthyl)indole-2-carboxamide (méthode G).

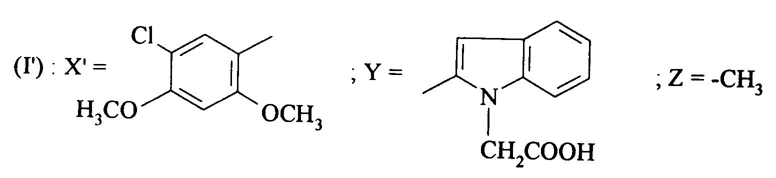

On agite à TA pendant une nuit un mélange de 1 g du composé obtenu à la PREPARATION IV. D, 0,96 g de l'acide N-(*ter*t-butoxycarbonylméthyl)indole-2-carboxylique, 1,6 g de BOP, 0,49 ml de triéthylamine dans 6 ml de DMF. On verse le mélange réactionnel sur une solution tampon pH = 2, filtre le précipité formé et lave ce dernier à l'eau. On reprend le précipité au DCM, lave par une solution tampon pH = 2, par une solution saturée d'hydrogénocarbonate de sodium, sèche sur sulfate de magnésium et évapore sous vide. On chromatographie sur silice en éluant par le mélange DCM/MeOH (100/1,5 ; v/v). On reprend l'ester *tert*-butylique obtenu dans 10 ml de TFA et laisse 1 heure et 30 minutes sous agitation à TA. On évapore sous vide, reprend le résidu dans l'eau, filtre le précipité formé, lave à l'eau et sèche sous vide à l'étuve. On obtient 1,37 g du produit attendu, F = 167°C.

### EXEMPLE 10

N-[4-(2,5-diméthoxy-4-méthylphényl)thiazol-2-yl]-1(carboxyméthyl)indole-2-carboxamide (méthode H).

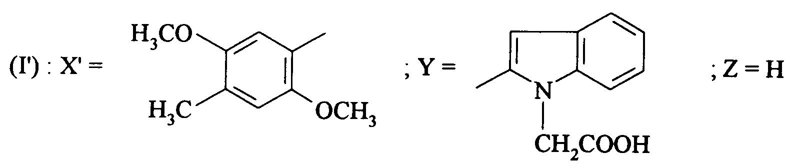

On agite à TA pendant 48 heures un mélange de 1,0 g de 2-amino-4-(2,5-diméthoxy-4-méthylphényl)thiazole, 1,09 g de l'acide N-(*tert*-butoxycarbonylméthyl)indole-2-carboxylique, 2,0 g de BOP, 0,55 ml de triéthylamine dans 5 ml de DMF. On verse le mélange réactionnel sur une solution tampon pH = 2, filtre le précipité formé et lave ce dernier à l'eau. On reprend le précipité au DCM, lave par une solution saturée d'hydrogénocarbonate de sodium, par une solution tampon de pH = 2, sèche sur sulfate de magnésium et évapore sous vide. On chromatographie sur silice en éluant par le mélange DCM/AcOEt (100/1,5 ; v/v). On reprend l'ester *tert*-butylique obtenu dans 10 ml de TFA et laisse 3 heures sous agitation à TA. On évapore sous vide, reprend le résidu par une solution de NaOH 2N, lave au DCM, acidifie la phase aqueuse par ajout d'HCl concentré, filtre le précipité formé et sèche sous vide à l'étuve. On obtient 1,4 g du produit attendu, F = 206°C.

### EXEMPLE 11

N-[4-(4-trifluorométhyl-2,6-diméthoxyphényl)thiazol-2-yl]-1H-indole-2-carboxamide (méthode F).

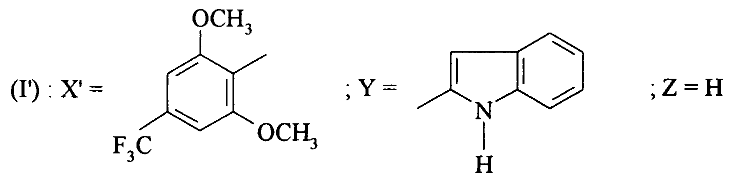

On agite à TA pendant 48 heures un mélange de 0,609 g de 2-amino-4-(4-trifluorométhyl-2,6-diméthoxyphényl)thiazole, 0,447 g d'acide N-acétylindole-2-carboxylique, 1,062 g de BOP, 0,243 g de triéthylamine dans 30 ml de DCM. On ajoute 100 ml d'eau, après décantation sèche la phase organique sur sulfate de sodium et évapore sous vide. On reprend le dérivé acétylé sur l'azote indolique obtenu dans 50 ml de MeOH, ajoute 2 g de carbonate de sodium et agite une nuit à TA. On évapore sous vide, reprend le résidu par 100 ml d'eau, extrait au DCM, sèche sur sulfate de sodium et évapore sous vide. On obtient 0,54 g du produit attendu après cristallisation dans le DCM, F > 260°C.

### EXEMPLE 12

N-[4-(4-trifluorométhyl-2,6-diméthoxyphényl)thiazol-2-yl]-1-(*tert*-butoxycarbonylméthyl)indole-2-carboxamide (méthode I).

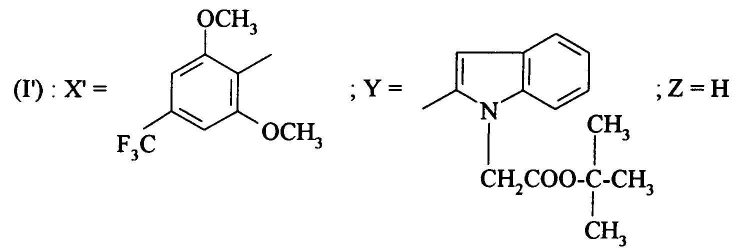

On agite à TA pendant 24 heures un mélange de 0,609 g de 2-amino-4-(4-trifluorométhyl-2,6-diméthoxyphényl)thiazole, 0,606 g d'acide N-(*tert*-butoxycarbonylméthyl)indole-2-carboxylique, 1,062 g de BOP et 0,243 g de triéthylamine dans 30 ml de DCM. Puis on ajoute 50 ml d'eau, après décantation on sèche la phase organique sur sulfate de sodium et évapore sous vide. On chromatographie sur silice H en éluant par le mélange DCM/AcOEt (100/5 ; v/v). On obtient 0,79 g du produit attendu après cristallisation dans l'éther, F =214-216°C.

### EXEMPLE 13

Trifluoroacétate de N-[4-(4-trifluorométhyl-2,6-diméthoxyphényl)thiazol-2-yl]-1-(carboxyméthyl)indole-2-carboxamide (méthode J).

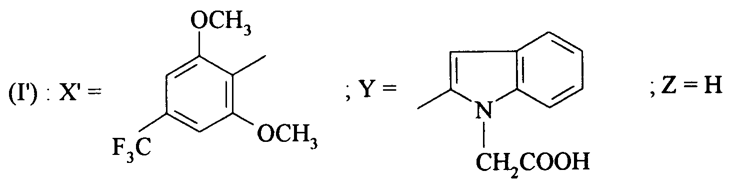

On refroidit à 10°C 20 ml de TFA, ajoute 0,5 g du composé obtenu à l'EXEMPLE 12 et laisse 3 heures sous agitation à 10°C. On évapore sous vide, reprend le résidu à l'eau, extrait à l'AcOEt, sèche sur sulfate de sodium et évapore sous vide. On obtient 0,47 g du produit attendu après cristallisation dans l'éther, F = 230-232°C.

En procédant selon les modes opératoires décrits ci-dessus, on prépare les composés de formule (I') décrits dans le TABLEAU IV ci-dessous.

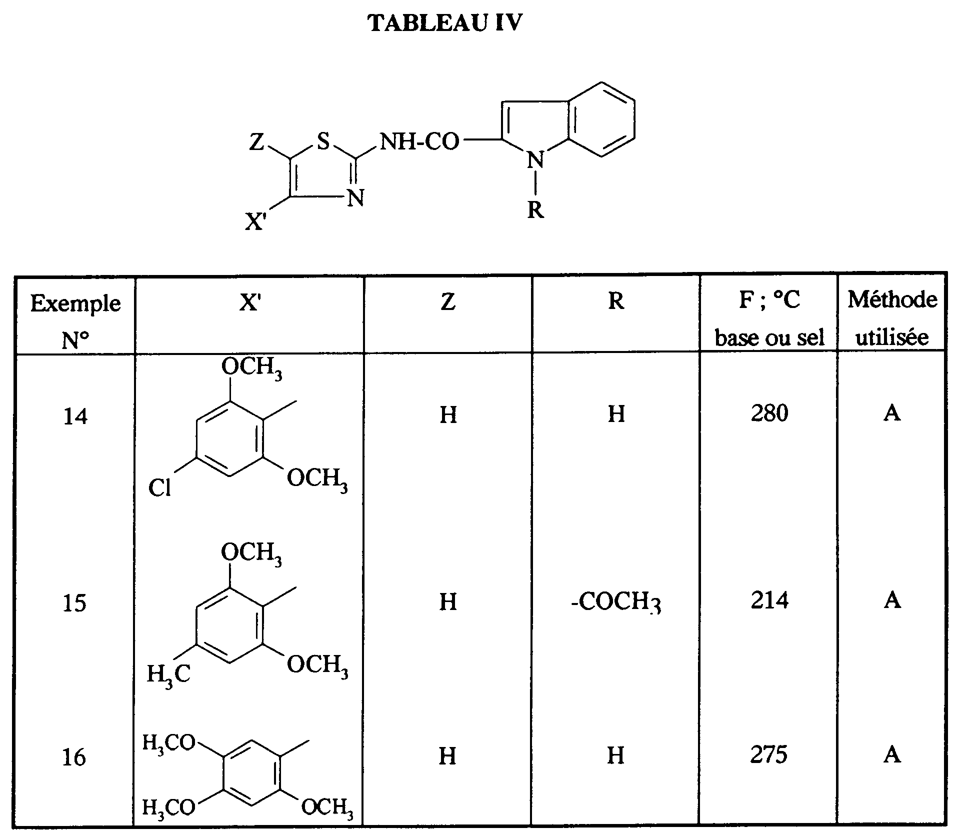

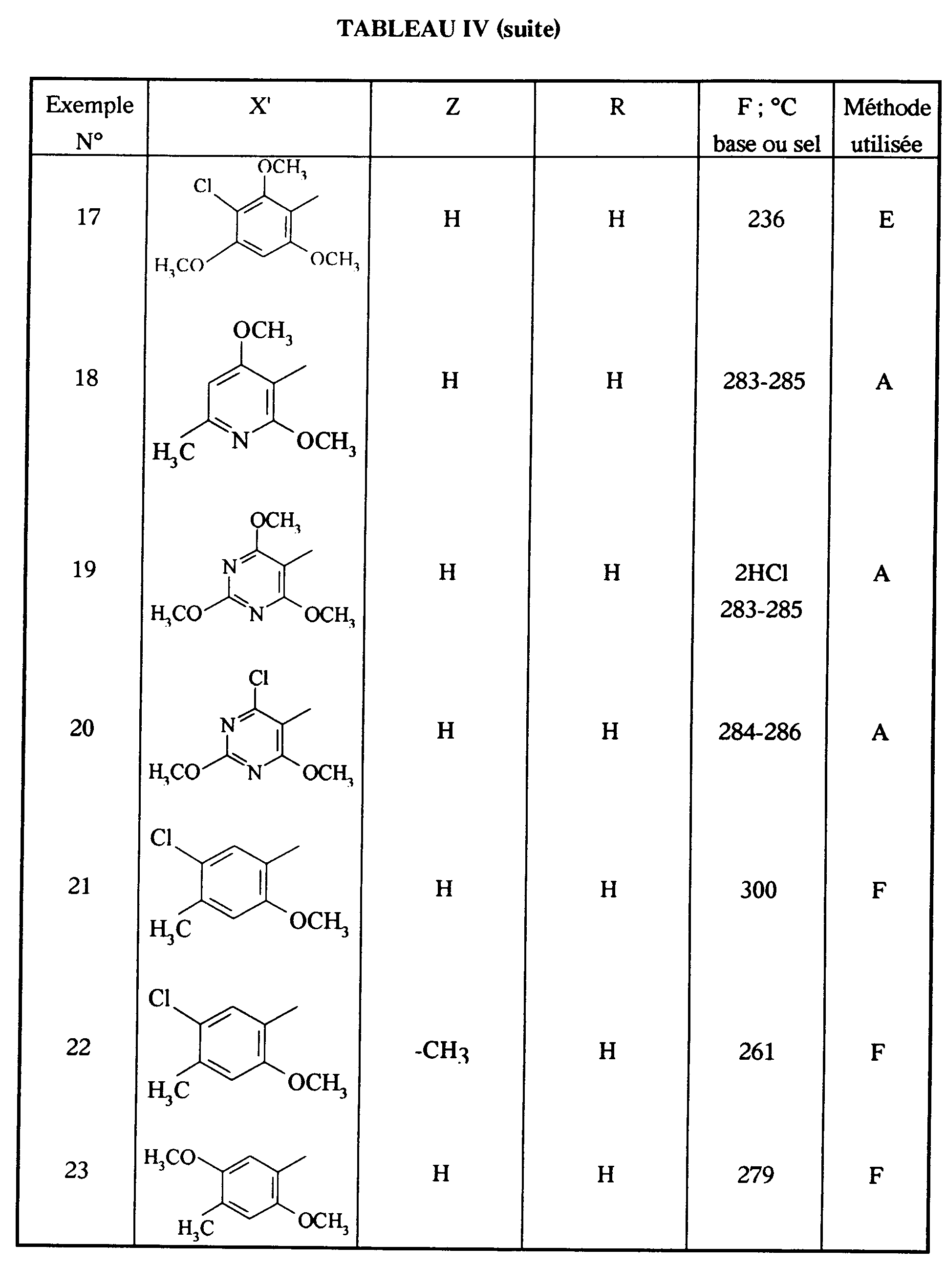

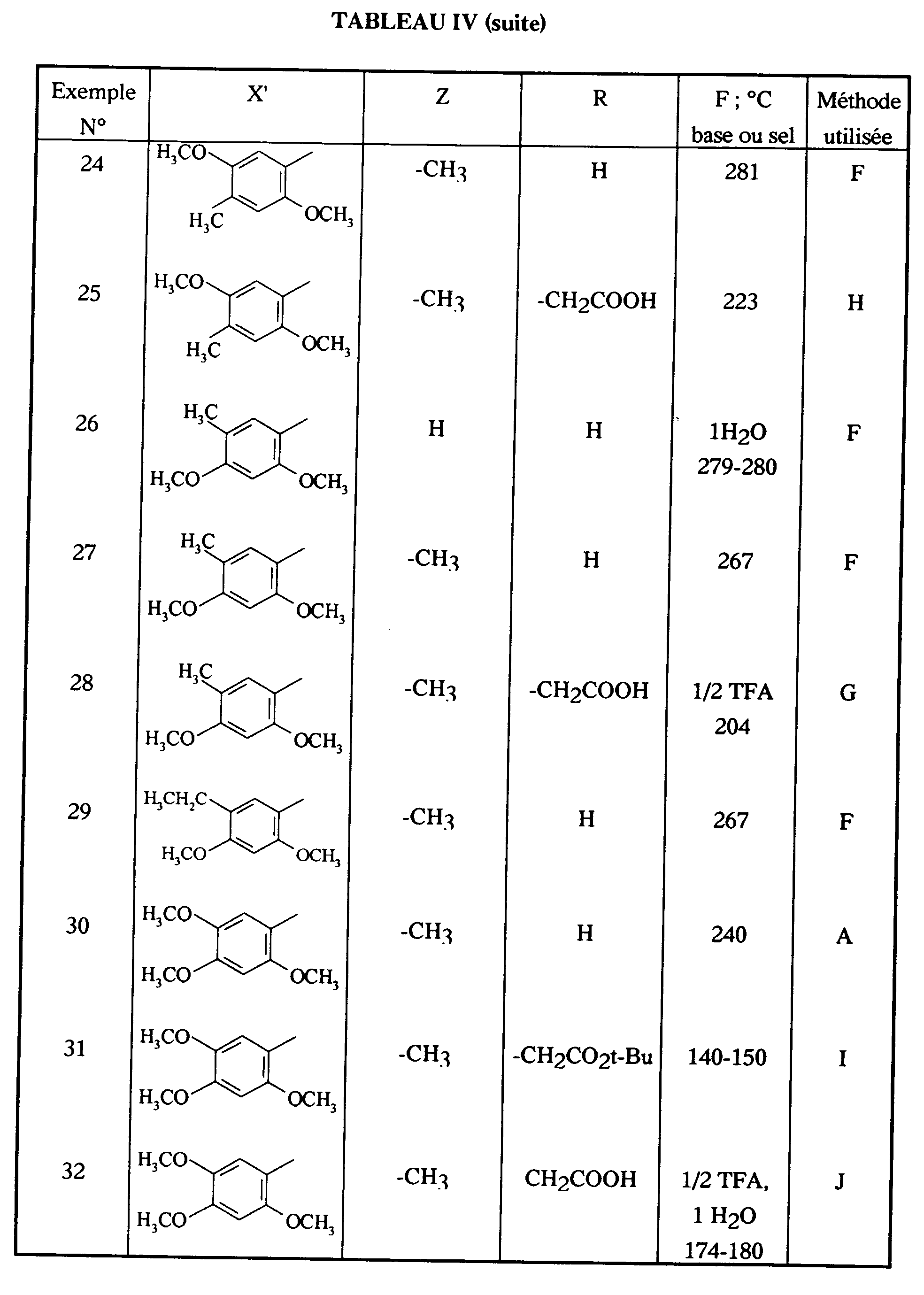

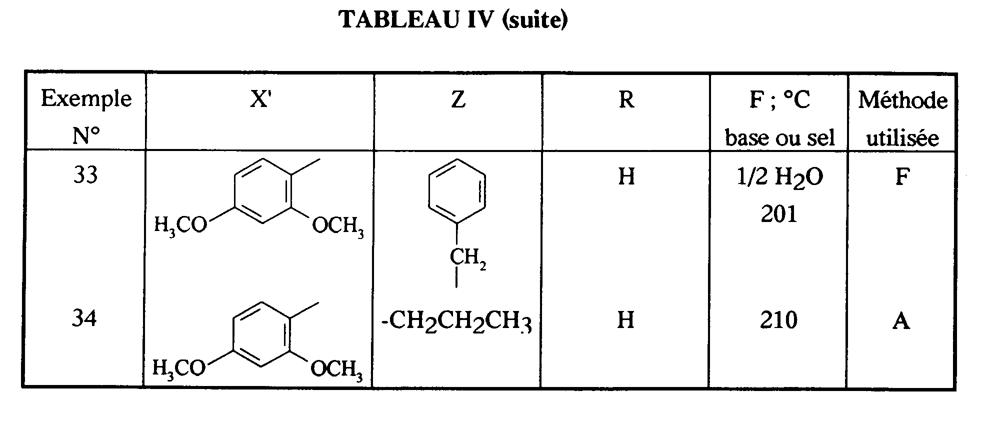

## Revendications

1. Utilisation d'un composé de formule: 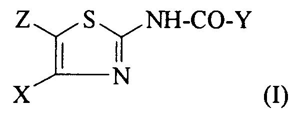 dans laquelle Y représente un groupe 3-quinoléinyle ou un groupe 2-indolyle de formule : 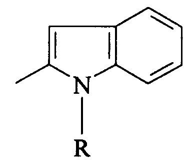 dans laquelle :
- R est l'hydrogène, un groupe acétyle ou un groupe CH₂COOR', R' étant l'hydrogène ou un akyle en C₁-C₄ ;
- X est un radical (hétéro)aryle choisi parmi les groupes 4-chloro-2,6-diméthoxyphényle, 2,6-diméthoxy-4-méthylphényle, 2,4,5-triméthoxyphényle, 4-méthyl-2,3,6-triméthoxyphényle, 2,6-diméthoxy-4-éthylphényle, 2,4,6-triméthoxy-5-chlorophényle, 2,4,6-triméthoxy-3-pyridinyle, 2,4-diméthoxy-6-méthyl-3-pyridinyle, 6-chloro-2,4-diméthoxy-5-pyrimidinyle, 2,4,6-triméthoxy-5-pyrimidinyle, 5-chloro-2,4-diméthoxyphényle, 5-chloro-2-méthoxy-4-méthylphényle, 2,5-diméthoxy-4-méthylphényle, 4-trifluorométhyl-2,6-diméthoxyphényle, 2,4-diméthoxy-5-méthylphényle, 5-éthyl-2,4-diméthoxyphényle, 2,4-diméthoxyphényle ;
- Z représente H, un alkyle en C₁-C₄ ou un benzyle ;
avec la limitation que Z est obligatoirement l'hydrogène, lorsque X est un radical phényle substitué simultanément aux positions 2 et 6 ou lorsque X est un radical 3-pyridinyle substitué simultanément aux positions 2 et 4 ou lorsque X est un radical 5-pyrimidinyle substitué simultanément aux positions 4 et 6 ;
ainsi que de ses sels pharmaceutiquement acceptables et de ses solvates
pour la préparation de médicaments destinés à combattre les maladies dont le traitement nécessite une stimulation par agonisme total ou partiel des récepteurs de la cholécystokinine.

2. Un composé de formule: 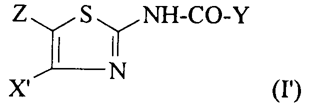 dans laquelle Y représente un groupe 3-quinoléinyle (a) ou un groupe 2-indolyle (b) de formule : 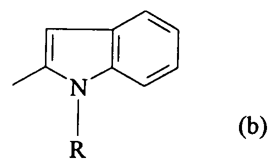 dans laquelle :
- R est l'hydrogène, un groupe acétyle ou un groupe CH₂COOR', R' étant l'hydrogène ou un alkyle en C₁-C₄ ;
- X' est un radical (hétéro)aryle choisi parmi les groupes 4-chloro-2,6-diméthoxyphényle, 2,6-diméthoxy-4-méthylphényle, 2,4,5-triméthoxyphényle, 4-méthyl-2,3,6-triméthoxyphényle, 2,4,6-triméthoxy-5-chlorophényle, 2,4,6-triméthoxy-3-pyridinyle, 2,4,6-triméthoxy-5-pyrimidinyle, 2,4-diméthoxy-6-méthyl-3-pyridinyle, 6-chloro-2,4-diméthoxy-5-pyrimidinyle, 5-chloro-2,4-diméthoxyphényle, 5-chloro-2-méthoxy-4-méthylphényle, 2,5-diméthoxy-4-méthylphényle, 4-trifluorométhyl-2,6-diméthoxyphényle, 2,4-diméthoxy-5-méthylphényle, 5-éthyl-2,4-diméthoxyphényle ;
- Z représente H, un alkyle en C₁-C₄ ou un benzyle ;
avec la limitation que Z représente H lorsque X' est un radical phényle substitué simultanément aux positions 2 et 6 ou lorsque X' est un radical 3-pyridinyle substitué simultanément aux positions 2 et 4 ou lorsque X' est un radical 5-pyrimidinyle substitué simultanément aux positions 4 et 6 ;
ainsi que ses sels et ses solvates.

3. Un composé selon la revendication 2 de formule (I'), dans laquelle Y représente un groupe (b) où R est l'hydrogène ou un groupe CH₂COOH, ainsi que ses sels pharmaceutiquement acceptables et ses solvates.

4. Un composé selon la revendication 2 de formule (I'), dans laquelle Z représente un hydrogène ou un méthyle, ainsi que ses sels pharmaceutiquement acceptables et ses solvates.

5. Un composé selon la revendication 2 de formule (I'), dans laquelle Y représente un radical (b) où R est l'hydrogène ou un groupe CH₂COOH, Z représente un hydrogène ou un méthyle et X' représente un radical aryle choisi parmi les groupes 5-chloro-2,4-diméthoxyphényle,4-chloro-2,6-diméthoxyphényle,5-chloro-2-méthoxy-4-méthylphényle, 2,6-diméthoxy-4-méthylphényle, 2,4-diméthoxy-5-méthylphényle et 2,4,5-triméthoxyphényle (Z étant obligatoirement un hydrogène lorsque X' représente un groupe 4-chloro-2,6-diméthoxyphényle ou 2,6-diméthoxy-4-méthylphényle), ses sels pharmaceutiquement acceptables et ses solvates.

6. Composé selon la revendication 2 choisi parmi
le N-[4-(4-chloro-2,6-diméthoxyphényl)thiazol-2-yl]-1-(carboxyméthyl)indole-2-carboxamide,
le chlorhydrate du N-[4-(4-chloro-2,6-diméthoxyphényl)thiazol-2-yl]-1-(carboxyméthyl)indole-2-carboxamide,
le N-[4-(5-chloro-2,4-diméthoxyphényl)thiazol-2-yl]-1H-indole-2-carboxamide,
le N-[4-(5-chloro-2,4-diméthoxyphényl)-5-méthylthiazol-2-yl]-1H-indole-2-carboxamide,
le N-[4-(5-chloro-2,4-diméthoxyphényl)-5-méthylthiazol-2-yl]-1-(carboxyméthyl)-indole-2-carboxamide,
le trifluoroacétate du N-[4-(5-chloro-2,4-diméthoxyphényl)-5-méthylthiazol-2-yl]-1-(carboxyméthyl)-indole-2-carboxamide,
le N-[4-(5-chloro-2-méthoxy-4-méthylphényl)thiazol-2-yl]-1H-indole-2-carboxamide,
le N-[4-(2,6-diméthoxy-4-méthylphényl)thiazol-2-yl]-1H-indole-2-carboxamide,
le chlorhydrate monohydrate du N-[4-(2,6-diméthoxy-4-méthylphényl)thiazol-2-yl]-1H-indole-2-carboxamide,
le N-[4-(2,4-diméthoxy-5-méthylphényl)-5-méthylthiazol-2-yl]-1H-indole-2-carboxamide,
le N-[4-(2,4,5-triméthoxyphenyl)-5-méthylthiazol-2-yl]-1H-indole-2-carboxamide, leurs sels et solvates pharmaceutiquement acceptables.

7. Procédé pour la préparation d'un composé de formule (I') selon la revendication 2, caractérisé en ce qu'on condense un acide de formule (II) :
Y'-COOH (II)
dans laquelle Y' représente un radical 3-quinoléinyle (a) 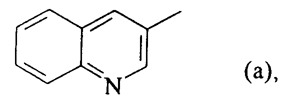 un radical 2-indolyle (b°) 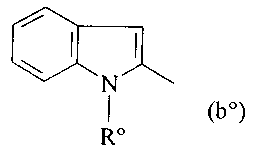 ou un radical 2-indolinyle (c°) 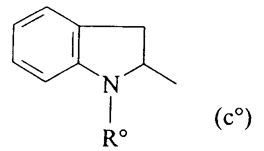 R° étant groupe N-protecteur ou un groupe CH₂COOR'', où R'' est un alkyle en C₁-C₄ ; ou bien un dérivé fonctionnel dudit acide (II) sur un 2-aminothiazole de formule : 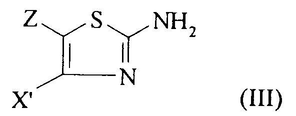 dans laquelle X' et Z sont tels que définis à la revendication 2, en présence d'une base pour obtenir un composé de formule (I'') : 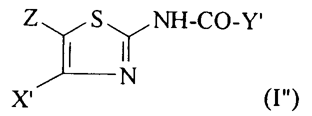 dans laquelle X' et Z sont tels que définis à la revendication 2 et Y' est un des radicaux (a), (b°) ou (c°) tels que définis ci-dessus, puis,
lorsque dans le composé (I''), Y' est un radical (b°), on soumet éventuellement le produit ainsi obtenu de formule (I''b°) : 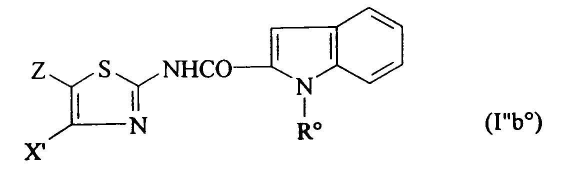 à une N-déprotection, à une saponification ou à une hydrolyse acide;
lorsque dans le composé (I''), Y' est un radical (c°), on soumet le produit ainsi obtenu de formule : 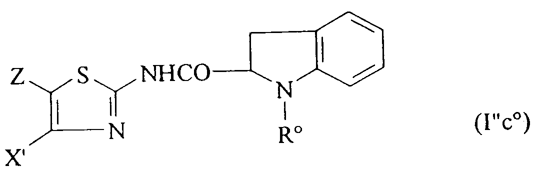 à une déshydrogénation, éventuellement précédée d'une N-déprotection, d'une saponification ou d'une hydrolyse acide pour obtenir un composé de formule (I')
dans laquelle Y est un radical (b) où R est l'hydrogène ou un groupe CH₂COOR', X', Z et R' étant tels que définis à la revendication 2, et on isole le produit de formule (I') tel quel ou sous forme d'un de ses solvates ou de ses sels.

8. Un composé de formule : 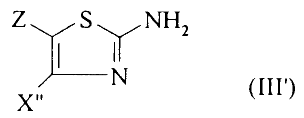 dans laquelle :
- X'' représente un radical(hétéro)aryle choisi parmi les groupes 4-chloro-2,6-diméthoxyphényle, 2,6-diméthoxy-4-méthylphényle, 4-méthyl-2,3,6-triméthoxyphényle, 2,4,6-triméthoxy-5-chlorophényle, 2,4,6-triméthoxy-3-pyridinyle, 2,4,6-triméthoxy-5-pyrimidinyle, 2,4-diméthoxy-6-méthyl-3-pyridinyle, 6-chloro-2,4-diméthoxy-5-pyrimidinyle, 5-chloro-2,4-diméthoxyphényle, 5-chloro-2-méthoxy-4-méthylphényle, 2,5-diméthoxy-4-méthyl-phényle, 4-trifluorométhyl-2,6-diméthoxyphényle, 2,4-diméthoxy-5-méthylphényle, 5-éthyl-2,4-diméthoxyphényle ;
- Z représente H, un alkyle en C₁-C₄ ou un benzyle
avec la limitation que Z représente H lorsque X'' est un radical phényle substitué simultanément aux positions 2 et 6 ou lorsque X'' est un radical 3-pyridinyle substitué simultanément aux positions 2 et 4 ou lorsque X'' est un radical 5-pyrimidinyle substitué simultanément aux positions 4 et 6.

9. Un composé selon la revendication 8, choisi parmi
le 2-amino-4-(4-chloro-2,6-diméthoxyphényl)thiazole,
le 2-amino-4-(5-chloro-2,4-diméthoxyphényl)thiazole,
le 2-amino-4-(5-chloro-2,4-diméthoxyphényl)-5-méthylthiazole,
le 2-amino-4-(5-chloro-2-méthoxy-4-méthylphényl)thiazole,
le 2-amino-4-(2,6-diméthoxy-4-méthylphényl)thiazole,
le 2-amino-4-(2,4-diméthoxy-5-méthylphényl)-5-méthylthiazole.

10. Composition pharmaceutique, caractérisée en ce qu'elle comprend une dose thérapeutiquement efficace d'au moins un composé selon l'une des revendications 2 à 6, en association avec au moins un excipient.

11. Composition pharmaceutique sous forme d'unité de dosage, comprenant une dose thérapeutiquement efficace d'au moins un composé selon l'une des revendications 2 à 6, en association avec au moins un excipient.

12. Composition pharmaceutique comprenant de 0,05 à 100 mg d'un composé selon l'une des revendications 2 à 6 en association avec au moins un excipient.

13. Utilisation selon la revendication 1 d'un composé de formule (I) dans laquelle
Y et R ont la même signification qu'à la revendication 1;
X est un radical (hétéro)aryle choisi parmi les groupes 4-chloro-2,6-diméthoxyphényle, 2,6-diméthoxy-4-méthylphényle, 2,4,5-triméthoxyphényle, 4-méthyl-2,3,6-triméthoxyphényle, 2,6-diméthoxy-4-éthyl-phényle, 2,4,6-triméthoxy-5-chlorophényle, 2,4,6-triméthoxy-3-pyridinyle, 2,4,6-triméthoxy-5-pyrimidinyle, 2,4-diméthoxy-6-méthyl-3-pyridinyl,
6-chloro-2,4-diméthoxy-5-pyrimidinyl;
Z représente H ou méthyle avec la limitation que Z représente H lorsque X' est un radical phényle substitué simultanément en position 2 et 6 ou lorsque X' est un radical 3-pyridinyle substitué simultanément aux positions 2 et 4 ou 2 et 6 ou lorsque X' est un radical 5-pyrimidinyle substitué simultanément aux positions 4 et 6 ou 2 et 6, ainsi que ses solvates et ses sels pharmaceutiquement acceptables.

14. Composé selon la revendication 2 de formule (I') dans laquelle
Y et R ont la même signification qu'à la revendication 1 et X' est un groupe (hétéro)aryle choisi parmi 4-chloro-2,6-diméthoxyphényle, 2,6-diméthoxy-4-méthylphényle, 2,4,5-triméthoxyphényle, 4-méthyl-2,3,6-triméthoxyphényle, 2,4,6-triméthoxy-5-chlorophényle, 2,4,6-triméthoxy-3-pyridinyle, 2,4,6-triméthoxy-5-pyrimidinyle, 2,4-diméthoxy-6-méthyl-3-pyridinyl, 6-chloro-2,4-diméthoxy-5-pyrimidinyl;
Z représente H ou méthyle avec la limitation que Z représente H lorsque X' est un radical phényle substitué simultanément en position 2 et 6 ou lorsque X' est un radical 3-pyridinyle substitué simultanément aux positions 2 et 4 ou 2 et 6 ou X' est un radical 5-pyrimidinyle substitué simultanément aux positions 4 et 6 ou 2 et 6, ainsi que ses solvates et ses sels pharmaceutiquement acceptables.
